Europäisches Patentamt

European Patent Office

Office européen des brevets

(11) Publication number: **0 369 567**
**A2**

# EUROPEAN PATENT APPLICATION

(21) Application number: **89302314.3**

(51) Int. Cl.5: **C12N 15/13, A61K 39/395**

(22) Date of filing: **08.03.89**

(30) Priority: **17.11.88 US 274106**

(43) Date of publication of application:
**23.05.90 Bulletin 90/21**

(84) Designated Contracting States:
**AT BE CH DE ES FR GB GR IT LI LU NL SE**

(71) Applicant: **HYBRITECH INCORPORATED**
**11095 Torreyana Road**
**San Diego California 92126(US)**

(72) Inventor: **Johnson, Mary Jacqueline**
**16231 El Camino Real**
**Rancho Sante Fe California 92067(US)**

(74) Representative: **Hudson, Christopher Mark et al**
**Erl Wood Manor**
**Windlesham Surrey GU20 6PH(GB)**

(54) **Chimeric antibodies directed against metal chelates.**

(57) The present invention discloses novel chimeric monoclonal antibodies, directed against metal chelates of EDTA derivatives, having chelate-specific variable regions of defined amino acid sequences. DNA constructs for the eight and heavy chain variable regions comprising the novel antibodies of the invention are provided. Eukaryotic host cells capable of expression of the chimeric antibodies and comprising the novel chimeric antibody-encoding DNA constructs are also provided.

EP 0 369 567 A2

## CHIMERIC ANTIBODIES DIRECTED AGAINST METAL CHELATES

Monoclonal antibodies are becoming increasingly important for both in vitro application in immunoassays and for the in vivo diagnosis and treatment of disease. Monoclonal antibodies which are directed against metal chelates of aminobenzyl derivatives of EDTA are particularly useful for the in vivo imaging and treatment of tumors associated with certain carcinomas, including colorectal and breast carcinomas.

Most available monoclonal antibodies, however, are derived from murine, i.e., mouse, hybridomas. The in vitro application of murine antibodies in immunoassays presents potential problems associated with false positive results which are attributable to the reaction of serum components with murine immunoglobulins. More importantly though, the in vivo application of murine antibodies in human medicine is often limited due to their inherent immunogenicity. The administration of murine antibodies will, in many patients, induce an immune response which results in a gradual decline in the efficacy of the antibodies during multiple dose regimens. This decrease in efficacy is attributable, at least in part, to the rapid clearance from circulation or alteration of pharmacokinetic properties of murine antibodies by the patient's immune response. The immunogenicity associated with murine monoclonal antibodies, therefore, precludes multiple dose administrations over an extended period of time and substantially impacts their potential clinical value.

It has been suggested that the use of human monoclonal antibodies for clinical application would overcome the limitations associated with the use of murine monoclonal antibodies. However, human monoclonal antibodies having the desired specificity and affinity for antigens and haptens are technically difficult to prepare.

Chimeric antibodies, in which the binding or variable regions of antibodies derived from one species are combined with the constant regions of antibodies derived from a different species, have been constructed by recombinant DNA methodology. Chimeric antibodies are described, for example, in European Patent Publication 173494; Shaw, et al., J. Immun., 138:4534 (1987), Sun, L.K. et al., Proc. Natl. Acad. Sci. USA, 84:214-218 (1987); Neuberger, M.S. et al., Nature, 314:268 (1985), Boulianne, G.L. et al. Nature, 312:643-646 (1984); and Morrison, S.L. et al. Proc. Natl. Acad. Sci. USA, 81:6851-6855 (1984). Typically, the variable region of a murine antibody is joined with the constant region of a human antibody. It is expected that as such chimeric antibodies are largely human in composition, they will be substantially less immunogenic than murine antibodies. Accordingly, chimeric monoclonal antibodies are highly desirable for in vivo application.

While the general concept of chimeric antibodies has been described, there exists a need for human therapeutic and tumor-imaging purposes for the development of novel chimeric monoclonal antibodies having specificity for predetermined metal chelate haptens of interest, particularly metal complexes of para-aminobenzyl EDTA derivates and variable regions of defined amino acid sequences. Further, there exists a need for the development of DNA constructs having defined DNA coding sequences for light and heavy chain variable regions which comprise novel chimeric antibodies, and which are capable of expressing these novel chimeric proteins in eukayotic cells. The present invention meets these needs.

This invention is directed to chimeric monoclonal antibodies, and the recombinant DNA compounds and vectors encoding such antibodies, as set forth below in the Detailed Description and the Claims. These chimeric antibodies have specificity for metal chelates of EDTA (ethylenediaminetetraacetate) complexes, (referred to herein as "metal hapten complex", "complex hapten", "metal hapten" or simply "chelate"), preferably wherein the chelate is L- or D- (para)-aminobenzyl EDTA, and more preferably, the indium (III) chelates of L- or D-(para)-aminobenzyl EDTA. The metal complexes include the therapeutic and imaging radionuclides of the metal ion. The variable regions for the present antibodies are derived from the murine antibody CHA 255.5, as it is described in D.T. Reardan et al., Nature, 316, pp. 265-268 (1985), and C.F. Meares et al., U.S. Patent No. 4,722,892, issued February 2, 1988, herein incorporated by reference. The present antibodies also encompass variations of the amino acid sequence in the CHA 255.5 variable regions, when such variants have approximately the same binding specificity as the murine CHA 255.5 variable regions do for the metal complex haptens.

For purposes of the present invention, as disclosed and claimed herein, the following terms are abbreviated as indicated below:

| Amino Acid | Three Letter Abbreviation |
|---|---|
| Alanine | Ala |
| Arginine | Arg |
| Asparagine | Asn |
| Aspartic acid | Asp |
| Cysteine | Cys |
| Glutamic Acid | Glu |
| Glutamine | Gln |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophan | Trp |
| Tyrosine | Tyr |
| Valine | Val |

| Nucleic Acid | One Letter Abbreviation |
|---|---|
| Deoxyadenyl | A |
| Deoxyguanyl | G |
| Deoxycytidyl | C |
| Thymidyl | T |

FIGURE 1 - Restriction site and function map of plasmids pMLCH-1 and pGCHAK with legends referring to plasmid pMLCH1dB. (For the purpose of this disclosure, all Figures ara not drawn exactly to scale.)

FIGURE 2 - Restriction site and function map of plasmid pHKF-1.

FIGURE 3 - Restriction site and function map of plasmids pUCVHInc-1A and pHG1Z with restriction sites.

FIGURE 4 - Restriction site and function map of plasmids pHG1-CHA and pNCHAG1 with restriction sites.

FIGURE 5 - Restriction site and function map of plasmids p19HAN and p19HANCH.

FIGURE 6 - Restriction site and function map of plasmid pGCHAK-3.

One aspect of the present invention is a recombinant DNA compound that comprises DNA encoding a chimeric antibody light chain ("DNA light chain compound") comprising a chelate-specific variable region derived from a first mammalian species and a constant region derived from a second and different mammalian species said light chain variable region having an amino acid sequence comprising:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

Also included in the invention is the recombinant DNA compound encoding for the DNA light chain compound ("light chain coding strand") wherein the coding strand for the light chain variable region comprises:

3

CAG GCT GTT GTG ACT CAG GAA TCT GCA CTC ACC ACA TCA CCT GGT GAA ACA GTC ACA CTC
ACA TGT CGC TCA AGT ACT GGG GCT GTT ACA ACT AGT AAC TAT GCC AAC TGG GTC CAA GAA
AAA CCA GAT CAT TTA TTC ACT GGT CTA ATA GGT GGT ACC AAT AAC CGG GCT CCA GGT GTT
CCT GCC AGA TTC TCA GGC TCC CTG ATT GGA GAC AAG GCT GCC CTC ACC ATC ACA GGG GCA
CAG ACT GAA GAT GAG GCA AGA TAT TTC TGT GCT CTA TGG TAC AGC AAC CTC TGG GTA TTC
GGT GGA GGA ACC AAA CTG ACT GTC CTA GGG

(Due to the complimentary nature of DNA base pairing, the sequence of a double-stranded DNA molecule is sufficient to determine the sequence of the opposing strand. Thus, in this disclosure only the coding strand of the DNA sequences are given.)

A further variant of the above DNA light chain compound is a recombinant DNA compound that comprises DNA encoding a chimeric antibody light chain comprising a chelate-specific variable region and signal peptide derived from a first mammalian species and a constant region derived from a second and different mammalian species, said light chain variable region and signal peptide having an amino sequence comprising:

Met Ala Trp Ile Ser Leu Ile Leu Ser Leu Leu Ala Leu Ser Ser Gly Ala Ile Ser Gln Ala Val Val Thr Gln Glu
Ser Ala Leu Thr Thr Ser Pro Gly Glu Thr Val Thr Leu Thr Cys Arg Ser Ser Thr Gly Ala Val Thr Thr Ser Asn
Tyr Ala Asn Trp Val Gln Glu Lys Pro Asp His Leu Phe Thr Gly Leu Ile Gly Gly Thr Asn Asn Arg Ala Pro Gly
Val Pro Ala Arg Phe Ser Gly Ser Leu Ile Gly Asp Lys Ala Ala Leu Thr Ile Thr Gly Ala Gln Thr Glu Asp Glu
Ala Arg Tyr Phe Cys Ala Leu Trp Tyr Ser Asn Leu Trp Val Phe Gly Gly Gly Thr Lys Leu Thr Val Leu Gly

Also claimed as part of this invention is the recombinant DNA compound which is the coding strand for the above variant of the DNA light chain compound wherein the coding strand for the signal peptide and the light chain variable region comprises:
ATG GCC TGG ATT TCA CTT ATA CTC TCT CTC CTG GCT CTC AGC TCA GGT GCC ATT TCC CAG
GCT GTT GTG ACT CAG GAA TCT GCA CTC ACC ACA TCA CCT GGT GAA ACA GTC ACA CTC ACT
TGT CGC TCA AGT ACT GGG GCT GTT ACA ACT AGT AAC TAT GCC AAC TGG GTC CAA GAA AAA
CCA GAT CAT TTA TTC ACT GGT CTA ATA GGT GGT ACC AAT AAC CGG GCT CCA GGT GTT CCT
GCC AGA TTC TCA GGC TCC CTG ATT GGA GAC AAG GCT GCC CTC ACC ATC ACA GGG GCA CAG
ACT GAA GAT GAG GCA AGA TAT TTC TGT GCT CTA TGG TAC AGC AAC CTC TGG GTA TTC GGT
GGA GGA ACC AAA CTG ACT GTC CTA GGG

Preferred embodiments of the above DNA light chain compound, the light chain coding strand or the above signal-peptide containing variants thereof are:

a) a recombinant DNA light chain compound, coding strand or signal peptide-containing variants thereof wherein the chelate-specific light chain variable region derived from a first mammalian species is derived from a murine hybridoma;

b) a recombinant DNA vector of a) wherein the constant region derived from a second and different mammalian species is derived from a human source;

c) a recombinant DNA vector of b) wherein the constant region derived from a second and different mammalian species is derived from a human source and said constant region taken from a human kappa light chain;

d) a recombinant DNA vector of c), which further comprises a promoter for the chelate-specific light chain variable region derived from a murine hybridoma that is the same promoter driving the expression of that gene in the murine hybridoma;

e) a recombinant DNA vector of d) wherein the vector is suitable for use as a cloning vector in a prokaryotic organism;

f) a recombinant DNA vector of d) which further comprises a promoter and translational activating sequence positioned to drive expression of said DNA in a cell of a eukaryotic organism;

g) a recombinant DNA vector of f) wherein the vector is derived from a pSV2gpt or pSV2neo plasmid; and

h) a recombinant DNA vector of g) wherin the vector is plasmid pGCHAK or pNCHAK.

In the context of the present invention, the term "chimeric antibody" refers to an antibody comprising a chelate-specific variable region derived from a first mammalian species and a constant region derived from a second and different mammalian species.
Accordingly, the chimeric antibodies of the invention are the product of fused genes encoding for a variable region having specificity for chelates and a constant region having predetermined structural and physiological properties not naturally associated with the variable region.

A second aspect of the invention is a recombinant DNA compound that comprises DNA encoding a chimeric antibody heavy chain comprising an chelate-specific variable region derived from a first mammalian species and a constant region derived from a second and different mammalian species, said heavy

4

chain variable region having an amino acid sequence comprising:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Ley Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

(The above aspect is referred to as the "DNA heavy chain compound".)

Also included as part of the DNA heavy chain compound is a recombinant DNA ("heavy chain coding strand") compound wherein the coding strand for the heavy chain variable region comprises:

GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC

A further variant of the above DNA heavy chain compound is a recombinant DNA compound that comprises DNA encoding a chimeric antibody heavy chain comprising a chelate-specific heavy chain variable region and signal peptide derived from a first mammalian species an a constant region derived from a second and different mammalian species, said heavy chain variable region having an amino acid sequence comprising:

Met Ser Phe Gly Leu Ser Leu Ile Phe Leu Val Leu Ile Leu Lys Gly Val Gln Cys Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

Also claimed as part of this invention is a recombinant DNA compound of the above variant of the DNA heavy chain compound wherein the coding strand for the signal peptide and the heavy chain variable region comprises:

ATG AGC TTT GGG CTG AGC TTG ATT TTC CTT GTC CTA ATT TTA AAA GGT GTC CAG TGT GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC

Preferred embodiments of the above DNA heavy chain compound, the heavy chain coding strand, or the signal peptide-containing variants thereof are:

a) a recombinant DNA vector that comprises the DNA sequence of the above DNA heavy chain compound, coding strand or signal peptide-containing variants wherein the chelate-specific variable region derived from a first mammalian species is derived from a murine hybridoma;

b) a recombinant DNA vector of a) wherein the constant region derived from a second and different mammalian species is derived from a human source;

c) a recombinant DNA vector of b) wherein the constant region derived from a second and different mammalian species is derived from a human source and said constant region is the constant region taken from a human IgG1 heavy chain;

d) a recombinant DNA vector of c) wherein the vector is suitable for use as a cloning vector in a prokaryotic organism;

e) a recombinant DNA vector of d) wherein the vector is plasmid pHG1-CHA;

f) a recombinant DNA vector of c) wherein the vector further comprises a promoter and translational activating sequence postioned to drive expression of said DNA in a cell of a eukaryotic system;

g) a recombinant DNA vector of f) wherein the vector is derived from plasmids pSV2neo or pSV2gpt; and

h) a recombinant DNA vector of g) wherein the vector is plasmid pNCHAGI or pGCHAG1.

A third aspect of the instant invention is a chimeric monoclonal antibody comprising a chelate-specific variable region derived from a first mammalian species and a constant region derived from a second and different mammalian species, wherein the variable light chain region having an amino acid sequence comprises:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

A variant of the above chelate-specific light chain chimeric monoclonal antibody has the variable light chain region and the corresponding signal peptide having an amino sequence comprising:

Met Ala Trp Ile Ser Leu Ile Leu Ser Leu Leu Ala Leu Ser Ser Gly Ala Ile Ser Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

Preferred embodiments of the above chelate-specific light chain chimeric monoclonal antibody or the variant with the corresponding signal peptide thereof are:

a) a chelate-specific light chain chimeric monoclonal antibody wherein the variable light chain region is derived from a murine hybridoma;

b) a chimeric monoclonal antibody of a) wherein the constant light chain region is derived from a human source; and

c) a chimeric monoclonal antibody of b) wherein the constant light chain region is derived from a human source and is a human kappa constant region.

Yet another aspect of the invention is a chimeric monoclonal antibody comprising a chelate-specific heavy chain variable region derived from a first mammalian species and a constant region derived form a second and different mammalian species, the variable heavy chain region having an amino acid sequence comprising: Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

A variant of the above chelate-specific heavy chain chimeric monoclonal antibody has the variable heavy chain region and the corresponding signal peptide having an amino acid sequence comprising:

Met Ser Phe Gly Leu Ser Leu Ile Phe Leu Val Leu Ile Leu Lys Gly Val Gln Cys Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

Preferred embodiments of the above chelate-specific heavy chain variable region chimeric monoclonal antibody or variant with the corresponding signal peptide include:

a) a chelate-specific heavy chain variable region chimeric monolonal antibody wherein the variable heavy chain region is derived from a murine hybridoma;

b) a chimeric monoclonal antibody of a) wherein the constant heavy chain region is derived from a human source; and

c) a chimeric monoclonal antibody of b) wherein the constant heavy chain region is derived from a human source and is from the human IgG1 constant region.

The invention also embraces a chimeric monoclonal antibody comprising a chelate-specific variable region derived from a first mammalian species and a constant region derived from a second and different mammalian species, the variable light chain region having an amino acid sequence comprising:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and the variable heavy chain region having an amino acid sequence comprising:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala

A variant of the above chelate-specific heavy and light chain antibody is also included in the invention and has the variable light chain region and the corresponding signal peptide having an amino acid sequence comprising:

Met Ala Trp Ile Ser Leu Ile Leu Ser Leu Leu Ala Leu Ser Ser Gly Ala Ile Ser Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and the variable heavy chain region and the corresponding signal peptide having an amino acid sequence comprising:

Met Ser Phe Gly Leu Ser Leu Ile Phe Leu Val Leu Ile Leu Lys Gly Val Gln Cys Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Thr Val Ser Ala

Preferred embodiments of the above chelate-specific heavy and light chain chimeric monoclonal antibody and it s variant with heavy and light chain signal peptide sequences include:

a) a chelate-specific heavy and light chain chimeric monoclonal antibody wherein the variable regions are derived from a murine hybridoma;

b) a chimeric monoclonal antibody of a) wherein the constant regions are derived from a human source;

c) a chimeric monoclonal antibody of b) wherein the light chain constant region is a human kappa constant region;

d) a chimeric monoclonal antibody of b) wherein the heavy chain constant region is a constant region of human IgG1; and

e) a chimeric monoclonal antibody of b) wherein the light chain constant region is a human kappa constant region and the heavy chain constant region is a human IgG1 constant region.

As used herein, the term "variable region" refers to the region of light and heavy chain antibody molecules which is capable of binding chelates. The amino acid sequence of the variable region will vary depending upon the chelate determinant to which it binds and the manner in which it recognizes that chelate determinant. This diversity is related to the exon sequences in the genes which encode for the variable region as well as variants in the chelates.

The term "constant region" as used herein, refers to the region of light and heavy chain antibody molecules which provides structural stability and other biological functions but is not involved with binding chelates. The amino acid sequence and corresponding exon sequences in the genes of the constant region will be dependent upon the species from which it is derived; however, variations in the amino acid sequence will be relatively limited for particular constant regions within a species.

The disclosed DNA sequences for the signal peptides include a translational start signal, i.e., a DNA sequence that initiates translation of functional polypeptides. Those skilled in the art will recognize that, as the leader peptide does not function in the binding of chelates, DNA sequences encoding for alternative eukaryotic leader peptides may be suitably utilized in the invention. Accordingly, the present invention is not limited to the use of any particular eukaryotic signal peptide. Finally, a further aspect of the invention is the light and heavy chain signal peptides disclosed in the variants and the DNA compounds coding therefor.

Further, it will be appreciated by those skilled in the art that the first DNA coding sequences comprising the DNA constructs of the invention may be modified, for example, by site-directed mutagenesis to provide DNA constructs which are substantially equivalent. These modified DNA coding sequences are included in the invention provided they are capable of being translated into substantially the same chimeric polypeptides as described herein. The use of site-directed mutagenesis may, in certain cases, modify the affinity of the resulting chimeric polypeptides for chelates.

The first DNA coding sequences of the DNA constructs of the present invention are preferably derived from the genomic DNA of a murine hybridoma expressing monoclonal antibody directed against chelates. The murine hybridoma, designated as CHA255.5 expressing antibody having the desired specificity and affinity for metal, especially indium III chelates of EDTA derivatives as described in the C.F. Meares et al., U.S. Patent No. 4,722,892, is particularly preferred for use. However, the variable light and heavy chain regions may be derived from other mammalian species e.g., lapine, caprine, equine, bovine, and non-human primates, provided the DNA sequences, and resulting amino acid sequences upon translation, have substantially the same affinity to the sequences disclosed by the invention.

7

Genomic DNA for use in the invention can be obtained and cloned by conventional techniques and in a variety of ways. Such techniques are described in Basic Methods in Molecular Biology, edited by L.G. Davis, M.D. Dibner and J.F. Battey, Elsevier, New York (1986); Feder, J., et al., Am. J. Hum. Genetics, 37:635-649 (1985); and Steffer, D. and Weinberg, R.A., Cell, 15:1003-1010 (1978). For example, hybridoma cellular DNA may be isolated by standard procedures, the genomic DNA fragmented into restriction fragments by restriction endonucleases, and the resulting fragments cloned into suitable recombinant DNA cloning vectors and screened with radiolabeled or enzymatically labeled probes for the presence of the DNA sequences disclosed herein. As used herein, the term "restriction fragment" refers to any linear DNA sequence generated by the action of one or more restriction endonuclease enzymes. The term "recombinant DNA cloning vector", as used herein, refers to any autonomously replicating agent, including but not limited to, plasmids and phages, comprising a DNA molecule to which one or more additional DNA segments can be or have been added. The DNA sequences obtained from genomic DNA may also include intervening sequences or introns which do not code for polypeptides; these sequences can be subsequently modified by nucleotide deletion or substitution by standard procedures. See, for example, Kramer. W. et al., Nucleic Acids Res., 12:9441 (1984); and Kunkel, T.A. Proc. Nat. Acad. Sci. USA, 82:488 (1985).

The first DNA sequences of the DNA constructs of the invention, encoding for polypeptides which are variable light and heavy chain regions of chimeric antibodies, can also be obtained from cDNA. Procedures for obtaining and cloning cDNA are well known and described by Okayama, H. and Berg, P., Mol. Cell Biol., 2:161 (1982); and Gubler, U. and Hoffman, B.J. Gene, 25:263 (1983). Accordingly, cDNA can be cloned by standard procedures and the resulting clones screened with a suitable probe for cDNA coding for the variable regions defined herein. After the desired clones have been isolated the cDNA may be manipulated in essentially the same manner as genomic DNA

Alternatively, the first DNA sequences, containing the requisite genetic information for variable light and heavy chain region specificity for chelates, may be synthetically prepared using con ventional procedures. Techniques for synthesizing DNA sequences are described by Sinha, N.D. et al. Nucleic Acid Res. 12:4359 (1984); and Beaucage S.L. and Caruthers, M.H., Tetrahedron Letters, 20:1859 (1981). The first DNA sequences encoding for light and heavy chain variable regions may, therefore, be synthesized from nucleotide monomers using conventional DNA synthesis technology to yield a DNA coding sequence capable of being translated into substantially the same polypeptides as described herein. This synthetic DNA sequence does not need to be identical to the cloned gene if degenerate codons are substituted for the original codons provided they code for the same amino acid upon translation.

The second DNA sequences of the DNA constructs of the invention, encoding for constant light and heavy chain regions of chimeric antibodies, can be cloned from genomic DNA and cDNA, or prepared synthetically. Preferably, the second DNA sequences encoding for constant region polypeptides are derived from human lymphocytes, e.g. human peripheral blood lymphocytes. The use of DNA sequences coding for human constant regions is expected to result in the production of chimeric light and heavy chain polypeptides which minimize immunogenicity. Particularly preferred for use are the DNA sequences derived from the human light (kappa) chain and the human heavy (gamma) chain genes, including the various isotypic classes (e.g., IgM, IgA), subclasses (e g., IgA1, IgA2), types (e.g., kappa or lambda), subgroups (V kappa I, VHI, etc.) and allotypes (such as the Gm groups), particularly the gamma-1 gene, described by Heiter, P.A. et al., Cell 22:197-207 (1980) and Takahashi, N. et al., Cell, 29:671-679 (1982). However, the present invention is not limited to particular second DNA sequences encoding for human constant regions for light and heavy chain chimeric polypeptides. Additionally, those skilled in the art will appreciate that, provided the species selected is different than the species from which the variable region is derived, the constant region genes may be derived from other mammalian species. For example, the constant region may be derived from species such as lapine, caprine, bovine, equine, porcine and non-human primates, depending upon whether the antibody will have in vitro or in vivo application.

The recombinant DNA techniques necessary to prepare the chimeric DNA constructs of the invention, and incorporate these constructs into appropriate recombinant DNA cloning vectors and recombinant DNA expression vectors, are now well known in the art and described by numerous references. See, e.g., Eukarytic Viral Vectors, edited by Y. Gluzman, Cold Spring Harbor Laboratories Publications, Cold Spring Harbor New York (1982); Eukaryotic Transcription, edited by Y. Gluzman, Cold Spring Harbor, New York (1985); Sequence Specificitv in Transcription & Translation, edited by R. Calendar and L. Gold, Allan R. Liss, Inc., New York, (1985); Maximizing Gene Expression, edited by W. Reznikoff and L. Gold, Butterworths, New York (1986); Mammalian Cell Technology, edited by W.G. Thilly, Butterworths, New York (1986); J. Sambrook and M.J. Gething, Focus (Bethesda Research Laboratories/Life Technologies Inc.) 10, #3. pp. 41-48 (1988). As used herein, the term "recombinant DNA expression vector" refers to any recombinant DNA cloning vector which includes a promoter sequence for directing transcription of DNA into RNA, and appropriate regulatory sequences to initiate and terminate transcription of RNA and translation of RNA into polypeptides. Furthermore, the term "recombinant DNA vector" refers to either a recombinant DNA cloning vector or recombinant DNA expression vector, as those terms are defined herein.

In accordance with the invention, the DNA constructs encoding for light and heavy chain chimeric polypeptides are introduced into appropriate eukaryotic host cells as part of an expression vector. These constructs can be contained on a single eukaryotic expression vector or maintained separately, with separate expression vectors each comprising a single chimeric gene construct. For expression of the chimeric polypeptides, however, it will be necessary to include transcriptional and translational regulatory sequences which are functional in the selected eukaryotic host cells. Accordingly, the chimeric genes can be isolated on large DNA fragments containing 5′ and 3′ untranslated regions as well as intronic sequences comprising homologous regulatory regions, e.g., promoters, enhancers, and transcriptional terminators and polyadenylate addition sites ("poly A sites"), all of which function within eukaryotic host cells. As used herein the terms "promoter" and "enhancer" refer to DNA sequences that direct transcription of DNA into RNA, and sequences that enhance transcription of DNA into RNA, respectively. The term "transcriptional terminator," as used herein, refers to a DNA sequence that terminates transcription of DNA into RNA. The term "poly A site" refers to a DNA sequence which designates location of a poly-A tail sequence attachment. Alternatively, the chimeric genes can be recombined with a variety of heterologous regulatory regions such as the well known SV40 and Herpes TK viral sequences, which contain viral promoters, enhancers, transcriptional terminators, and poly A sites. The chimeric gene constructs can also be combined with synthetic regulatory elements provided that these elements can function in eukaryotic host cells and are properly fused to the chimeric genes. cDNA clones or synthesized genes can also be combined with either homologous or heterologous regulatory sequences in order to be expressed as polypeptides. Skilled artisans will appreciate, therefore, that homologous, heterologous or synthetic regulatory regions may be used interchangeably for purposes of expression of the chimeric genes of the present invention.

A wide variety of recombinant expression vectors are well known and may be used in the invention. Preferably, the pSV2-type vectors are utilized. The pSV2-type vectors comprise segments of the SV40 genome that constitute a defined eukaryotic transcription unit and transform mammalian and other eukaryotic host cells by integrating into the host cell chromosomal DNA. A variety of plasmid pSV2-type vectors have been constructed (see, Eukaryotic Viral Vectors, edited by Y. Gluzman, Cold Spring Harbor Laboratory Publications, Cold Spring Harbor, New York, (1982)), such as plasmids pSV2-gpt, pSV2-neo, pSV2-dhfr, and pSV2-β-globin, in which the SV40 promoter directs transcription of an inserted gene. These vectors are available either from the American Type Culture Collection (ATCC) in Rockville, Maryland or from the Northern Regional Research Laboratory (NRRL) in Peoria, Illinois. Alternatively, expression vectors may be selected which can be maintained episomally, i.e., extrachromasomally, such as bovine papilloma virally-based expression vectors and Epstein Barr virus expression vectors. (See, Eukaryotic Viral Vectors, edited by Y. Gluzman, Cold Spring Harbor Laboratories Publications, Cold Spring Harbor, New York (1982); Papillomaviruses, edited by P M. Howley and T.R. Broker, Alan R. Liss, Inc., New York (1985); and Sugden, B. et al., Mol. Cell Biol., 5:410-413 (1985); Kioosis, D. et al., EMBO, 6:355-361 (1987); and J. Sambrook and M.J. Gething, Focus (Bethesda Research Laboratories/Life Technologies, Inc.) 10, #3, pp. 41-48 (1988).

Higher levels of expression can be attained by removing the SV40 enhancer from the expression vectors formed from the pSV2-class vectors. Nearly all genomic immunoglobulin genes contain enhancer sequences. The murine kappa variable region gene is found on plasmid pGCHAK in conjunction with an enhancer sequence. Skilled artisans will recognize that these enhancer sequences can be moved to many different sites on the expression vectors without changing the ability of transfected cells to produce these immunoglobulin chains. However, the removal of the SV40 enhancer from the expression vector pGCHAK leads to a marked increase in the levels of expression of the present antibodies from SP2/0 cells.

The CEM kappa promoter, found on plasmid pMLCE-10 (available from the ATCC under accession number ATCC 67639) is also useful to increase the levels of expression of heterologous immunoglobulin chains. Specifically, as described above, the lambda and gamma variable region-encoding genes from murine hybridoma CHA255 have been cloned. Plasmid pGCHAK-2, which comprised the murine lambda CHA variable region and human constant region genes driven by the CEM kappa promoter on an SV40 enhancer-containing vector was constructed according to the teaching of Example 14. Plasmid pGCHAK-3, which comprises the murine lambda CHA variable region and human constant region genes driven by the CEM kappa promoter on an SV40 enhancerless vector was also constructed according to the of Example 14. SP2/0 cells (available from the ATCC) transfected by either of these plasmids demonstrated levels of kappa expression which were much greated than cells transfected with vectors comprising the CHA lambda promoter. Indeed, those cells which were transfected with (SV40 enhancerless) vectors comprising the CEM kappa promoter driving the expression of the CHA chimeric kappa gene displayed a ten-fold increase in the levels of kappa expression above the levels demonstrated by cells transfected with (SV40 enhancer plus) vectors comprising the CHA chimeric genes driven by the CHA lambda promoter. Subsequent transfection of these high-producing cells with vectors containing chimeric heavy chain genes will give a corresponding increase in whole antibody production over cells using the CHA promoter in the kappa chain vector. Even greater levels of expression can be attained by subcloning the highest expressing cells, then growing these high expression subclones in well-defined, serum free media. From this it is apparent that the CEM kappa promoter has the specific ability to drive high levels of expression of both homologous (CEM) and heterologous (CHA or other) sequences. While the teaching of Example 14 demonstrates the presence of the CEM promoter on an approximately 2.2 kilobase ClaI/SspI restriction fragment, the CEM kappa promoter sequence can easily be derived by those skilled in the art. This phenomena is discussed in C.B. Beidler, M.J. Johnson, and J.R. Ludwig, Chimeric Antibodies Directed Against Human Cancinoembryonic Antigen, Attorney Docket No. H-7587A, U.S. Application No. 07/272,577, filed this even date, herein incorporated by reference. See also Mary J. Johnson et a1., U.S. Patent Application No. 07/165,856, filed March 9, 1988, herein incorporated by reference (European Patent Application No.89302312.7).

The eukaryotic host cells useful in the present invention are, preferably, hybridoma, myeloma, lymphoma or plasmacytoma cells. However, other eukaryotic host cells may be suitably utilized provided the mammalian host cells are capable of recognizing transcriptional and translational DNA sequences for expression of the chimeric genes; processing the leader peptide by cleavage of the leader sequence and secretion of the chimeric proteins; and optionally providing post-translational modifications of the chimeric proteins, e.g., glycosylation. Yet another aspect of the present invention is a transformed host cell, especially an SP2/0 host cell, that secretes the present chimeric antibodies. Preferred embodiments include transformed SP2/0 host cells transformed with any of the mammalian expression vectors disclosed in this specification.

Accordingly, the present invention provides eukaryotic host cells which are transformed by recombinant expression vectors comprising the chimeric gene constructs disclosed herein and which are capable of expressing the chimeric proteins of the invention. The term "transformation," as used herein, refers to the change in the genome of a host cell by introduction of DNA into the recipient cell. The tranformed host cells of the invention, therefore comprise at least one DNA construct comprising the chimeric light and heavy chain genes described herein, and transcriptional and translational sequences which are positioned in relation to the light and heavy chain-encoding DNA sequences to direct expression of these chimeric proteins.

The host cells used in the invention may be transfected in a variety of ways by standard procedures well known in the art. Among the standard transfection procedures which may be used are electroporation techniques protoplast fusion and calcium-phosphate precipitation techniques. Such techniques are generally described by Toneguzzo, F. et al., Mol. and Cell. Biol., 6:703-706 (1986); Chu, G., et al., Nucleic Acid Res., 15:1311-1325 (1987); Rice, D. et al., Proc. Natl. Acad. Sci. USA, 79:7862-7865 (1979) and; Oi, V., et al., Proc. Natl. Acad. Sci. USA, 80:825-829 (1983).

Preferably, the recombinant expression vectors comprising the chimeric constructs of the invention are transfected sequentially into host cells For example, the expression vectors comprising the chimeric light chain DNA constructs are first transfected into the host cells and those host cells expressing the chimeric light chain polypeptides are selected by standard procedures known in the art. See, e.g., Engvall, E. and Perlmann P., Immunochemistry, 8:871-874 (1971). The expression vectors comprising the chimeric heavy chain DNA constructs are, thereafter, transfected into the selected host cells. However, it will be recognized that both the chimeric light and heavy chain expression vectors can be introduced simultaneously into the host cells. Alternatively, both the chimeric gene constructs can be combined on a single expression vector for transfection into cells. Following transfection and selection, standard assays are performed for the detection of antibodies directed against chelates and identification of transformed cells expressing both the chimeric light and heavy chain genes defined by the invention.

Additionally, it is understood that minor modifications to the amino acid sequences of the chimeric polypeptides disclosed herein may result in variable regions which are substantially equivalent in the binding of chelates. These modifications are contemplated by the present invention provided the requisite specificity for chelates is retained.

The ability of the present chimeric monoclonal antibodies to recognize the chelate complex of a specific ion relative to a complex of the chelating agent with another ion permits the detection and separation of metal ions from solutions containing other metals when the metal of chioce forms a chelate. Furthermore, the present antibodies modify, often extending, the serum half-life of chelated radionuclides administered for therapy and imaging purposes. Also, in certain instances, the administration of the present chimeric monoclonal antibodies could result in enhanced uptake of radioactivity in the tumor relative to the rest of the body, the murine (i.e. non-chimeric) analogs of the present chimeric antibodies have exhibited such properties. See D.T. Reardan, C.F. Meares, D.A. Goodwin, M. McTigue, G.S. David, M.R. Stone, J.P. Leung, R.M. Bartholemew, and J.M. Frincke, Nature, 316, pp. 265-268 (1985). See also C.F. Meares et al., U.S. Patent No. 4,722,892, issued February 2, 1988, herein incorporated by reference.

The present chimeric monoclonal antibodies can be further used to form bufunctional antibodies, i.e. antibodies having dual specifities. The second specificity of this bifunctional antibody will typically be an antigen associated with a tumor, an infectious organism, or other disease states. Among such antigens may be tumor-associated antigens such as carcino-embryonic antigen (CEA), prostatic acid phosphatase (PAP), prostate specific antigen like. The bifunctional antibodies can be composed of a fragment or half molecule of the present anti-metal complex antibodies. The other "half" of the bifunctional antibody is a chimeric moiety of a different specificity.

The appropriate antibody fragments (Fab' or fragments thereof, for example) for subsequent joining can be made by methods well known in the art. See Generally, Parham, J. Immunology, 131, 2895 (1983); Lamoyi et al., J. Immunological Methods, 56, 235 (1983); Parham, id. 53, 133 (1982); and Matthew et al., id., 50 239 (1982). Antibody half molecules may be made from whole antibodies and reassembled chemically by methods well known in the art to give bifunctional antibodies fashioned from the instant anti-hapten antibodies. See, for example, K. Auditore-Hargreaves, U.S. Patent No. 4,479,895, issued October 30, 1984, and H.P. Paulus, U.S. Patent No. 4,444,878, issued April 24, 1984, both of which are herein incorporated by reference. For instance, the cross-linking agent bis(maleimido)methyl ether ("BMME") can be used to couple the fragments or half molecules.

Yet another method of fashioning bifunctional antibodies from the present hapten-specific chimeric antibodies is to transfect mammalian cells ( e.g. SP2/0) with a pair of mammalian expression vectors, one vector carrying the genes for the present heavy and light chains, the other vector containing the heavy and light chain genes for an antigen-specific half molecule. The cell lines can be transfected sequentially or simultaneously with the vectors by methods in the art, such as by electroporation. The construction of bifunctional antibodies by this method is disclosed in M.J. Johnson and J. Phelps, Bifunctional Chimeric Antibodies, Attorney Docket No. H-7622, U.S. Application No. 07/274,105, filed this even date, herein incorporated by reference (European Patent Application No.89302313.)5.

The methods of use for chimeric bifunctional antibodies parallel those for non-chimeric bifunctional antibodies. For examples of the bifunctional antibodies, see the above-referenced U.S. Patent Nos. 4,479,895 and 4,444,878. See also J. Martinis et al., U.S. Patent Application No. 367,784 filed April 12, 1982, (Int. Pub. No. WO83/03679) herein incorporated by reference.

The following examples and figures are offered for purposes of illustration of the present invention and are not intended to limit it in any way.

## EXAMPLES

Murine hybridoma cells, designated as CHA 255.5, were used in the examples to derive and clone genomic DNA for light and heavy chain variable regions. The cloned genomic DNA from murine hybridoma cHA 255.5, and human peripheral blood lymphocytes, were used for the construction of the chimeric genes.

Transfection of chimeric genes was accomplished by electroporation techniques, essentially as described by Toneguzzo, F., et al., Molecular and Cell. Biol., 6:703-706 (1986); and Chu, G. et al., Nucleic Acid Res., 15:1311-1325 (1987). The host cells SP2/0-Ag14 hybridoma cells were the recipients of the chimeric genes. The SP2/0-Ag14 hybridoma cells used are available from the American Type Culture Collection, Rockville, Maryland under the accession number ATCC CRL 1581.

In the following examples, the reader is directed to "Maniatis" and "Davis", which is a shortened notation for T. Maniatis et al., Molecular Cloning. A Laboratory Manual, Cold Spring Harbor Laboratory. Cold Spring Harbor, New York (1982) and Basic Methods in Molecular Biology, edited by L.G. Davis, M.D. Dibner and J.F. Battey, Elsevier, New York (1986); respectively.

The following examples also refer in an abbreviated manner to commercial supplies of enzymes, reagents and equipment. The following is a list of the locations of many of these manufacturers: Gibco-BRL ("Gibco" or "BRL" Gaithersberg, Md), New England Biolabs (Beverly, Mass.), Sigma Chemical Co. ("Sigma", St. Louis Mo.), Schleicher and Schwell (Keene, N.H.), and Stratagene, Incorporated ("Stratagene", La Jolla, Ca.), etc.

Restriction enzyme reactions were run using enzymes from BRL in the appropriate React buffer (BRL) at 37° C for approximately two hours, unless otherwise noted.

Unless otherwise noted, enzymes are obtained from BRL, Sigma or New England Biolabs.


EXAMPLE 1


Derivation of Mutant CHA255 Cell Lines


The purpose of the following experiments was to derive subclones from the CHA 255.5 hybridoma which no longer produce either the lambda 1 light or gamma 1 heavy immunogobulin protein chains. DNA from these cell lines could then be used in comparison to the parental cell line DNA in order to identify restriction fragments containing the specific CHA255 $V_H$ and $V_L$ genes.

CHA 255.5 cells were grown in Dulbecco's modified Eagle media H21 (Ontario Cancer Institute) supplemented with 9.0% horse serum (Bochnek Organic Chemicals), 0.2% Fetal Bovine Serum (Gibco) and $10^{-5}$ M 2-mercaptoethanol (Biorad).

Cells were then subcloned at 1 cell/well in 96 well tissue culture plates (Limbro). Supernatants from the subclones were analyzed using standard ELISA techniques as described by Engvall, E. and Perlmann, P., Immunochemistry 8, 871-874 (1971).

Essentially the analysis was done as described below. BSA benzyl EDTA was prepared by C.F. Meares et al., Analytical Biochemistry 142, pp. 68-78 (1984). Each molecule of BSA contained 5-10 chelates/molecule. The chelates were loaded with indium by adding 105% molar excess of InCl₃ over hapten to the BSA-chelate solution and allowed to incubate overnight in 0.13 M citrate pH 6. The solution was brought to volume with 10 mM Phosphate pH 7.2. Final concentration was 10 μg/ml.

Each well of a 96 well plate was then coated using 50 μl of In-BSA/well and allowed to dry overnight at 37° C. The plate was then washed thoroughly in $H_2O$ and PBS + 0.1% Tween (W/v). Fifty μl of supernatant fraction from each of the subclones were added to each well, and incubated for 2 hours at room temperature. Plates were again rinsed as detailed above. A goat anti-human kappa chain alkaline phosphatase conjugate (Tago #2496) was diluted 1:1000 in ths same medium as the supernatant material. 100 μl were added per well and allowed to incubate for 1 hour at room termperature. Plates were again rinsed as detailed above. The alkaline phosphatase substrate was prepared as per package instruction, 1 tablet per 3 ml of distilled $H_2O$ and 150 μl of this substrate was added to each well and allowed to incubate 30 minutes at 37° C. The reaction was quenched with 50 μl of 500 mM EDTA and then the absorbance was read at 405 nM. Those supernatants which did not give a signal were determined to be missing either the heavy or light chain genes as they did not bind the antigen present on the plate.

The cells and supernatant fractions correponding to the negative ELISA reading were then analyzed by standard SDS-PAGE in order to determine which chain was missing in the various mutant subclones. Supernatants were concentrated approximately 10 fold in an Amicon Stirred Cell apparatus with a YM30 filter. These concentrated supernatants along with concentrated supernatants from the parental hybridoma CHA255.5 were analyzed by modified Laemmli 10% SDS-PAGE reducing gels. Approximately 2.5 μg of Ig protein was analyzed per sample. Reduced samples included 0.1 M DTT in the SDS sample buffer. All samples were boiled for 5 minutes before loading. Low molecular weight protein standards (Pharmacia, Piscataway, N.J.) were routinely chromatographed on the gels. Protein gels were stained in methanol:acetic acid:$H_2O$ (4:1:6) and then visualized in order to determine which protein product was missing in the mutant subcloned cell lines.


Example 2


Plasmid pMLCH-1: Construction of Vector Containing Murine CHA 255.5 $V_L$ Lambda Gene


Part A: Isolation of DNA from CHA255.5 Murine Hybridoma and from Mutant Subclones Derived from CHA255.5

Genomic DNA from CHA 255.5 and the mutant subclone cell lines which had lost Ig expression were isolated by procedures essentially as described by Maniatis, pp. 280-281. Approximately 1 x 10⁸ hybridoma cells (parental or mutant) were collected by centrifugation (10′, 800 rym IEC clinical centifuge). The cells were washed 2x in PBS. The cells were then resuspended in 4 ml of TEN, i.e., a solution containing 10 mM Tris-HCl (pH 8.0), 2 mM EDTA, and 40 mM NaCl, and lysed by the addition of 200 μl of 10% SDS and 42 μl of Protease K solution (10 mg/ml) (Sigma). The cell suspension was incubated overnight at 37°C. DNA was isolated from the cell suspension by extracting the suspension extracted twice with a an equal volume of phenol:choloroform:iso-amyl alcohol (25:24:1 solution) and twice more with an equal volume of chloroform:iso-amyl alcohol (24:1 solution). The aqueous phases were combined and dialyzed against TE Buffer overnight. The DNA was treated for 2 hours with RNAse A at a concentration of 50μg/ml (Sigma), followed by Protease K to give a concentration of 200 μg/ml for one hour at 37°C. DNA was extracted again and dialyzed overnight as detailed above. Following dialysis, DNA was precip itated from the dialyzed extract by adding 1/10th volume of 2 M sodium acetate solution and 2 volumes of 95% ethanol and allowed to stand for 30 minutes at -20°C. The cold ethanol solution was centrifuged at 8000 RPM for 30 minutes. The resultant pellets of DNA were resuspended in a sufficient amount of TE buffer to yield a solution of 500 μg/ml of DNA, as determined by the solution's optical density at 260 nm (1 unit of Absorbance at 260 nm is equivalent to 50 μg/ml).


Part B: Restriction Fragment Analysis of CHA 255.5 Cellular DNA

In order to identify the size of the DNA fragment containing the rearranged Ig Gamma 1 CHA 255.5 heavy chain gene, restriction fragment analysis was done. The DNA used for probing the Southern blots was obtained from Dr. Phillip Tucker, University of Texas, Dallas, Texas. This plasmid contains murine Balb/C germline JH3 and JH4 sequences on a BamHI-EcoRI fragment. (The sequence for this fragment can be found in the NIH Data GeneBank, Accession #J00453).

DNA (20 μg) from the parental CHA255.5 and the mutant subcloned cell lines was digested with the restriction enzymes BamHI and XbaI (both singly and together) using 1-2 units/μg of DNA at 37°C overnight with the appropriate React Buffers. The digested DNA was then electrophoresed in a 1% agarose gel (Seakem) at 40 volts overnight using TBE buffer (89 mM Tris, 89 mM Borate, and 2 mM EDTA). The DNA was then transferred to nitrocellulose filters (Schleicher and Schuell) as described in Maniatis, pp. 383-386. The nitrocellulose filters were then hybridized (see Maniatis, pp. 387-389) with nick-translated (P.J. Rigby et al., J. Mol. Biology, 113, p237 (1977)) JH probes. From this comparison of DNA from cells that were either expressing or not expressing the CHA255.5 heavy chain immunoglobulin gene products it was determined that the CHA255.5 V$_H$ gene was located on an 8.6 kb BamHI fragment.

To determine the size of the restriction fragment containing the rearranged lambda light chain gene, a 0.5 kb DNA fragment containing the VL1-JL3 gene was used as a probe. The variable regions of the lambda family differ at only 11 nucleotides, therefore lambda probes will cross-hybridize to any of the lambda genes. This DNA was a gift from Dr. H. Murialdo of the University of Toronto, Department of Immunology. (The sequence of the V lambda genes is available from the NIH Data Genebank Accession #'s J00578 and J00579.)

Approximately the same procedure was used to identify the lambda-containing restriction fragment as was used to identify the heavy chain gene Products. The DNA was digested with a variety of restriction endonuclease. Following electrophoresis and transfer to nitrocellulose filters, the filters were probed with the V lambda probe. From these hybridizations, it was determined that the CHA255.5 specific light chain gene was contained within an 11 kb EcoRI fragment.


Part C: Construction of a Size Selected Library Containing the CHA255.5 V$_L$ Gene

CHA 255.5 cellular DNA was digested with EcoRI (1-2 units/μg) using React Buffer #2 in order to enrich for the correct size fragment containing the V$_L$ specific gene. The method of isolating size enriched DNA is described in Hozumi, N., G.E. Wu, H. Murialdo, L., Roberts, D. Vetter, W.L. Fife, M. Whiteley, and P. Sadowski, Proc. Natl. Acad. Sci. USA 81 p. 2484, (1981). To construct the size selected library 0.24 μg of this EcoRI-digested size-selected DNA was ligated to 2.5 micrograms of the phage vector predigested EMBL 4 DNA arms (Stratagene). These two DNA fragments were ligated together in 10X Ligase Buffer (0.5

μl of 500 mM Tris-HCl (pH8); 70 mM $MgCl_2$; 0.1 M in ATP; 10 mM dithiothreitol (dtt)) and T4 DNA ligase (2 units) at 4° C overnight. Following ligation, packaging was done using Gigapack In Vitro packaging system (Stratagene) in accordance with their product protocols and using the host E. coli strain P2.392 (Stratagene). Gigapack packaging mix (50 μl) and 5 μl of dilutions ($10^{-2}$ - $10^{-6}$) of the ligated phage wsre mixed together and incubated at 22° C for 2 hours. Phage dilution buffer (0.5 ml of a solution composed, per liter, of 5.8 g NaCl, 2 g $Mg_2SO_4$-$6H_2O$, 50 ml of 1 M Tris-HCl (pH 7.5), and 5 ml of 2% gelatin) was added. Phage were then titered using standard protocols as described in Maniatis, pp. 286-294. Following titration, the phage library was plated at a density of 20,000 plaques/100 mM plate using P2.392 cells (Stratagene) and incubated at 37° C overnight.

Part D: Isolation of a Phage Containing the CHA255.5-specific V Lambda Gene

To identify and isolate the CHA 255.5 lambda light chain gene, 3.0 x $10^5$ recombinant phage in E. coli P2.392 (Stratagene) were screened according to the protocol in Maniatis, pp. 312, and Grunstein and Hogness, Proc. Natl. Acad. Sci. 72 p. 3961 (1975). This was done using the murine V lambda probe derived from plasmids obtained from Dr. H. Murialdo, University of Toronto.

Nitrocellulose filter lifts were made and phage hybridizing to the V lambda region probe were selected for further analysis. DNA was isolated from the recombinant phage using procedures found in Maniatis, pp. 368-369 and digested with EcoRI using React Buffer #3. The size of the recombinant phage inserts was analyzed by gel electrophoresis. One recombinant was identified as having an 11 kb EcoRI fragment insert. DNA from this phage was further analyzed with a variety of restriction enzymes. This phage was designated φMLCH-lnc.

Part E: Final Construction of Recombinant Plasmid pMLCH-1 Containing the CHA 255.5 Murine V Lambda Gene

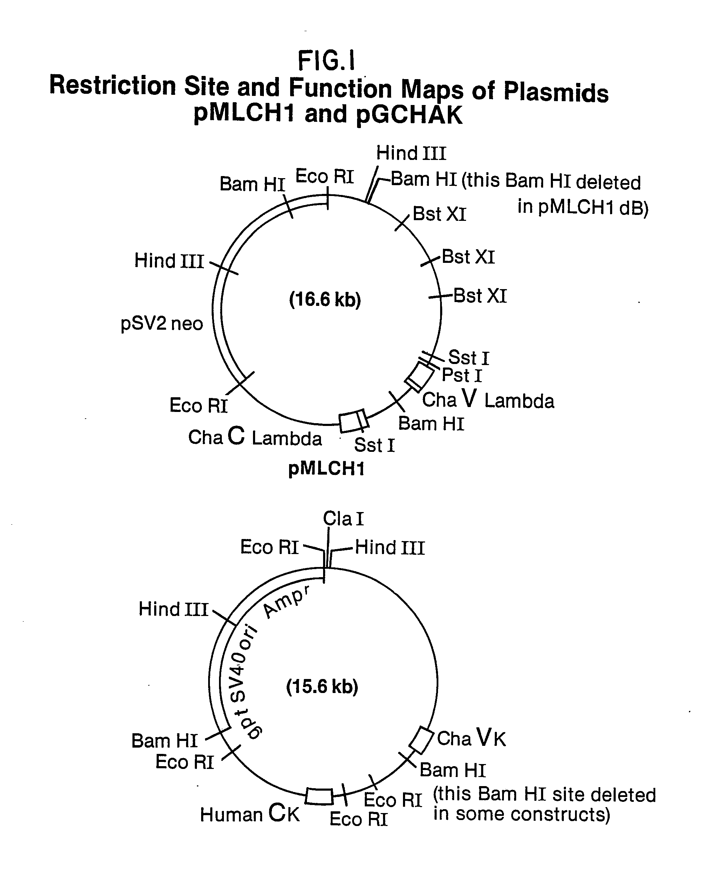

φMLCH-lnc DNA was isolated and a portion of (20 μg) of the isolated DNA were digested with EcoRI (1 unit/μg) using React Buffer #3 (220 μl). An 11 kb EcoRI fragment was isolated by electrophoresis of the EcoRI digested DNA in a 0.75% TBE agarose gel containing .5 μg/ml of ethidium bromide at 40 V overnight. Following visualization on a UV transparent light box, the 11 kb fragment was electrophoresed onto DEAE 81 paper (Schleicher and Schuell), followed by elution in 1 M NaCl and ethanol precipitation. The eluted fragment was then resuspended in 6 μl of TE buffer. The fragment was ligated to 50 ng of EcoRI-digested plasmid $pSV_2$neo, available from the American Type Culture Collection (ATCC Designation No. 37149) by using 1 μl (50 ng) of pSV2neo DNA, 6 μl (300 ng) of EcoRI digested 11 kb recombinant phage DNA, 1 μl of lOx Ligase Buffer and 1 μl of T4 DNA ligase. E. coli HB1O1 competent cells (BRL) were transformed using standard procedures. Specifically, HB101 cells were first thawed on ice; then 10μl of the ligation reaction mixture was mixed with 200μl of the HB101 cells and the resultant mixture was incubated on ice for 30 minutes. Following this, cells were heat shocked for 90 seconds at 42° C, then returned to ice for 2 minutes. One ml of LB broth (composed, per liter, of 10 g NaCl 5 g yeast extract, and 10 g tryptone) was added and the cells were incubated in a New Brunswick air shaker for one hour at 225 rpm, 37° C. A portion (two hundred μl) were then plated on LB-agarose with ampicillin (50 μg/ml) and incubated overnight at 37° C. Ampicillin resistant colonies were screened using colony screening methods as detailed in Maniatis pp. 312, and M. Grunstein and D. Hogness, Proc. Nat. Acad Sci., USA, 72, p. 3961 (1975). Again the Lambda murine probe was used to identify the resultant recombinant pSV2neo plasmid. This plasmid was designated as pMLCH-1 and was deposited under the Budapest Treaty with the Northern Regional Research Lab on November 14, 1988, where it is available as NRRL Deposit #B-18432. A restriction site and function map of plasmid pMLCH-1 is included in the drawings as Figure 1.

EXAMPLE 3

Plasmid pHKF-1: Construction of a Vector Containing a Human Constant Kappa Gene

## Part A: Isolation of Human DNA

Whole blood (30 ml samples) was obtained from individuals homozygous for the human gamma haplotypes fbn and azg. The samples were centrifuged and the top layer (buffy coat cells) were harvested from the blood samples by centrifugation at 2500 RPM in a Sorvall GLC-2B apparatus at 22°C. The buffy coat cells were removed with a Pasteur pipet and washed once with PBS. The cell pellets were resuspended in 500 $\mu$l of a solution composed of 10 mM Tris-HCl (pH 8.0) and 40 mM NaCl then lysed by the addition of 10% SDS (30 $\mu$l) and 20 mg/ml Protease K (6 $\mu$l) (Sigma). The sample was incubated 15 hours at 37°C. The DNA was extracted twice with an equal volume of phenol:chloroform:isoamyl alcohol mixture (25:24:1), twice more with chloroform:isoamyl alcohol mixture (24:1) and dialyzed against 10 mM Tris-HCl (pH 8.0) and 1 mM EDTA. The DNA was then treated for two hours with 50 $\mu$g/ml RNAse A (Sigma) followed by redigestion with 200 $\mu$g/ml Protesae K for 1 hour. The DNA was extracted and dialyzed as described above in Example 2B and the concentration determined by $OD_{260}$ to range from 100-200 mg/ml.

## Part B: Construction of a Human Genomic Phage Library

Human DNA of the fbn haplotype (210 $\mu$g as isolated in Example 3A) was partially digested with MboI by taking 10 $\mu$g of DNA in 11 $\mu$l of TE buffer, 20 $\mu$l of water, 15 $\mu$l of IOx Core Restriction Digest Buffer (500 mM Tris pH 8.0, 100 mM $MgCl_2$, 500 mM NaCl) and then aliquoted into tubes. Restriction enzyme MboI was added to each tube in units increasing from 0.0038 to 0.5 units/$\mu$l and placed at 37°C for one hour. Aliquots of the samples were then run on a 0.7% TBE (89 mM Tris, 89 mM borate and 2 mM EDTA) agarose gel and electrophoresed overnight at 40 volts. From the photograph of this gel it was determined which digestion fractions contained DNA in the correct molecular weight range (12-24 kb). Genomic DNA (200 $\mu$g) was then digested using the MboI units defined by the experiment described above (scaled up twenty times) with an hour incubation at 37°C. This DNA was used to construct the EMBL-3 library using EMBL-3 phage DNA (Stratagene) as described in the Stratagene protocol. The Stratagene protocol follows the techniques described in several laboratory manuals (e.g., Davis). Following isolation on sucrose gradients, the large molecular weight human DNA (12-24 kb) was ligated into the EMBL-3 phage arms in a reaction mixture consisting of 100 ng of DNA, 100 ng of preisolated BamHI-digested EMBL-3 arms, 0.5 $\mu$l of IOx Ligase Buffer (500 mM Tris-CHl, pH 8.9, 70 mM $MgCl_2$, 10 mM dtt) and T4 DNA ligase (2 units) and the reaction was maintained at 4°C overnight. Packaging was done using Stratagene's commercially available Gigapack-Gold In Vitro packaging system in accordance with their product protocols and using the host E. coli strain P2.392 supplied by Stratagene. 500 $\mu$l of Gigapack Gold packaging mix and 5$\mu$l of the ligated phage were incubated at 22°C for 2 hours. 0.5 ml of phage dilution buffer (per liter 5.8 g was NaCl, 2 g $Mg_2SO_4$-$6H_2O$, 50 ml 1 M Tris-HCl pH 7.5, 5 ml 2% gelatin was added. Phage were then titered using standard protocols as described in Davis, pp. 182-183 (1986). Following titration, the phage library was plated at a density of 20,000 plaques/100 mM plate using P2.392 cells and incubated at 37°C overnight.

## Part C: Final Construction of Recombinant Plasmid pHKF-1

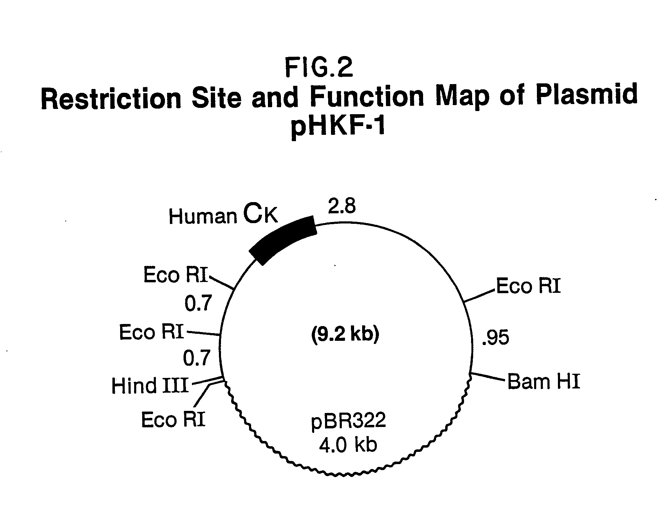

In order to isolate the human kappa constant region gene, the EMBL-3 library, as described in Example 3B, was screened with a human kappa probe supplied by Dr. P. Hieter, Johns Hopkins University, Baltimore, Maryland (the sequence of the probe is available from the NIH Data Base, accession number J00241.) A total of $5\times IO^5$ recombinant phage were screened as described in Example 2B. A single clone was isolated and designated $\varnothing$HKF-1. Fifteen $\mu$l of $\varnothing$HKF-1 DNA were digested simultaneously with BamHI and Hind III in React Buffer #3. A 5.2 kb fragment was isolated on DEAE 81 paper and ligated into the cloning vector pBR322 digested with BamHI and Hind III as described in Example 2E. Ampicillin resistant recombinant colonies were again identified using the human kappa probe; a single clone was isolated and designated pHKF-I. pHKF-1 was deposited under the Budapest Treaty with the American Type Culture Collection on March 1, 1988 and has the ATCC Deposit #67637. A restriction site and functon map of plasmid pHKF-1 is included in the drawings as Figure 2.

EXAMPLE 4

Plasmid pGCHAK - Mammalian Expression Vector Containing the Human Constant Region Kappa Gene and the Murine CHA 255.5 $V_L$ Lambda Gene

Part A: Construction of Plasmid pGCHA Including CHA 255.5 $V_L$ Lambda Gene in a Mammalian Expression Vector pSV₂gpt

1) Plasmid pMLCHldB

Several experimental steps were required in order to subclone the CHA 255.5 variable region gene into an expression vector. First, a BamHI site was deleted from the 5' end of the V lambda fragment containing the gene seen in Figure 1. This was accomplished using published protocols for 'fill-in' reactions (Maniatis, pp. 113-114). pMLCH1 (one μg) was partially digested for 3 minutes with BamHI using React Buffer #2. The BamHI ends were then made blunt (to delete the BamHI site) by adding 10 μl of a solution containing 5 mM concentration each of the 4 deoxyribonucleotides dTTP, dGTP, dCTP and dATP, 2 units of Klenow enzyme and a IOX buffer (0.5M Tris HCl pH 7.5; 0.1 M MgCl₂; 10 mM dithiothreitol) to give a total reaction mixture volume of 50 μl, as described in Maniatis, pp. 113-114. Following a 30 minute incubation of the reaction mixture, the DNA was extracted with a phenol-chloroform mixture, then precipitated with ethanol. The DNA was resuspended and self-ligated using T4 DNA ligase using standard ligation procedures analagous to that in Example 2E. Transformation was done using HB101 cells (BRL) and the resulting transformants were screened using plasmid mini-prep procedures (Maniatis, pp. 368-369) in order to identify a plasmid in which the correct BamHI site was successfully deleted. This construct was designated pMLCHldB. Figure 1 of the drawings describe the relation of plasmid pMLCHldB to plasmid pMLCH-1.

2) Plasmid pMLCH2

Plasmid DNA from pMLCHldB was isolated and used to construct pMLCH2. pMLCHldB (10 μg) was digested with the Hind III and BamHI using React Buffer #3. At the same time the vector pBR322 was digested with the same enzymes. Approximately 100 ng of the digested pBR322 DNA was mixed with 300 ng of the pMLCHldB digested DNA and ligated using T4 DNA ligase as described above. Again, the ligation products were introduced into HB101 bacterial cells (BRL) and the resulting transformants screened by mini-preparation (Maniatis) of the plasmid DNA in order to identify the correct subclone. The plasmid from the correct subclone was designated pMLCH2, and it contained the 5.75 kb CHA255.5-specific V lambda gene region inserted into the HindIII - BamHI site of pBR322.

3) Plasmid pSV2gpt-Cla

DNA from this plasmid was then used to construct the expression vector pGCHA. The expression vector pSV2gpt, available from the American Type Culture Collection, (ATCC Designation #37145) was used as the basis of the chimeric expression vector. pSV2gpt DNA (1 μg) was digested with the restriction enzyme EcoRI using 1 unit/μg of DNA in Reaction Buffer #3. The EcoRI ends were then made blunt by adding 10 μl of a solution 5 mM each of the 4 deoxyribonucleotides dTTP, dGTP, dCTP, and dATP, 2 units of Klenow enzyme and a IOX buffer (0.5 M Tris HCl pH 7.5; 0.1 M MgCl₂; 10 mM dithiothreitol) in a total volume of 50 μl, as described in Maniatis, pp. 113-114. The reaction went 30 minutes at room temperature and was followed by a ligation reaction in which phosphorylated ClaI linkers (2 μg) (New England BioLabs) were ligated to the 500 ng of EcoRI blunt-ended pSV₂gpt in order to create a new ClaI site for the future chimeric vector. The sequence ClaI linkers was d(pCATCGATG). Ligation reactions were carried out as described under Example 2E. Following ligation, excess linkers were removed by electrophoresis of the DNA and isolation of the linear pSV2gpt-Cla fragment onto DEAE 81 paper as in Example 2E. The isolated DNA was self-ligated using reagents and protocols described above. HB101 competent cells were transformed as in Example 2E and the ampicillin resistant colonies analyzed by restriction enzyme digestion.

The resulting vector, pSV2gpt-Cla, was then digested simultaneously with ClaI and BamHI restriction enzymes (1 unit/μg of DNA). The vector pMLCH2 was also digested with the same two restriction enzymes.

DNA from these digestions was then ligated together as described above in Example 2E, and then used to transform E. coli HB101. Again, plasmid DNA from the transformants was analyzed by restriction digestion and the expression plasmid pGCHA containing the gpt drug resistance gene mammalian expression vector and the CHA255.5 specific light chain lambda gene was identified.

### Part B. Construction of Plasmid pGCHAK

The eukaryotic expression vector containing the murine $V_L$ region fused to the human kappa gene was constructed using the vectors pGCHA and pHKF1. Plasmid pGCHA was digested with restriction enzyme BamHI and the approximately 11.15 kb fragment was isolated. Plasmid pHKF1 (available from the ATCC under the accession number 67637) was digested with HindIII in React buffer #3. The HindIII-digested plasmid was filled with Klenow, phosphorylated BamHI linkers (New England Biolabs) were added, then the vector was cut with BamHI, and the about 5.2 kb restriction fragment was isolated. This 5.2 kb BamHI fragment of plasmid pHKF-1 was ligated into the approximately 11.2 kb BamHI fragment of plasmid pGCHA to form plasmid pGCHAK. Plasmid pGCHAK comprises the gene that encodes the murine lambda CHA variable region joined to the gene which encodes the human kappa region. A restriction site and function map of this plasmid is included in the Drawings as Figure 1.

### EXAMPLE 5

### Plasmid pUCVHlnc-1A - construction of a Plasmid Containing the Murine CHA 255.5 Variable Heavy Chain Region

### Part A: Construction of a Size Selected Library Containing the CHA255.5 VH Gene

CHA 255.5 cellular DNA was digested with BamHI (1-2 units/$\mu$g) using React Buffer #2 in order to enrich for the correct size fragment containing the Vh specific gene. The method of isolating size-enriched DNA is described in Hozumi, N., G.E. Wu, H. Murialdo, L., Roberts, D. Vetter, W.L. Fife, M. Whiteley, and P. Sadowski, (1981) Proc. Natl. Acad. Sci. USA 81:2484. To construct the size-selected library, 0.24 $\mu$g of this BamHI-digested size-selected DNA was ligated to 2.5 micrograms of the phage vector (predigested) EMBL3 DNA arms (Stratagene). These two DNA fragments were ligated together in 10X Ligase Buffer (0.5 $\mu$l, 500 mM Tris-HCl pH 8; 70 mM MgCl$_2$; 10 mM dithiothreitol,) and T4 DNA ligase (2 units) at 4° C overnight. Following ligation, packaging was done with Gigapack In Vitro packaging system (Stratagene) in accordance with their product protocols and using the host E. coli strain P2.392 (Stratagene). Gigapack packaging mix (50 $\mu$l) and 5 $\mu$l of dilutions ($10^{-2}$ - $10^{-6}$) of the ligated phage were mixed together and incubated at 22° C for 2 hours. Phage dilution buffer (0.5 ml, a solution composed of 5.8 g NaCl, 2 g Mg$_2$SO$_4$·6H$_2$O, 50 ml 1 M Tris-HCl pH 7.5, and 5 ml 2% gelatin per liter) was added. Phage were then titered using standard protocols as described in Maniatis, p. 286. Following titration, the phage library was plated at a density of 20,000 plaques/100 mM plate using E. coli P2.392 (Stratagene) cells and incubated at 37° C overnight.

### Part B: Identification and Isolation of Recombinant Phage $\phi$MHCH-lnc Containing the Murine CHA255.5 VH Gene

For the CHA 255.5 heavy (gamma 1) chain 3.0 x $10^5$ recombinant phage in E. coli P2.392 (Stratagene) were screened according to the protocol in Maniatis, p. 320. The screening was done using the murine JH3-JH4 probe derived from plasmids obtained from Dr. Phillip Tucker, University of Texas, Dallas, Texas (NIH Data Genebank Accession No. J00453).

Nitrocellulose filter lifts were made and phage hybridizing to the JH region probe were selected for further analysis. DNA was isolated from the recombinant phage using procedures found in Maniatis, p. 365 and digested with BamHI using React Buffer #2. The size of the recombinant phage inserts was analyzed by gel electrophoresis. One recombinant was identified as having an 8.6 kb BamHI fragment. DNA from this phage was further analyzed with a variety of restriction enzymes and designated φMHCH-Inc.

Part C: Final Construction of Recombinant Plasmid pUCVHInc-1A containing CHA 255.5 Murine VH Gene

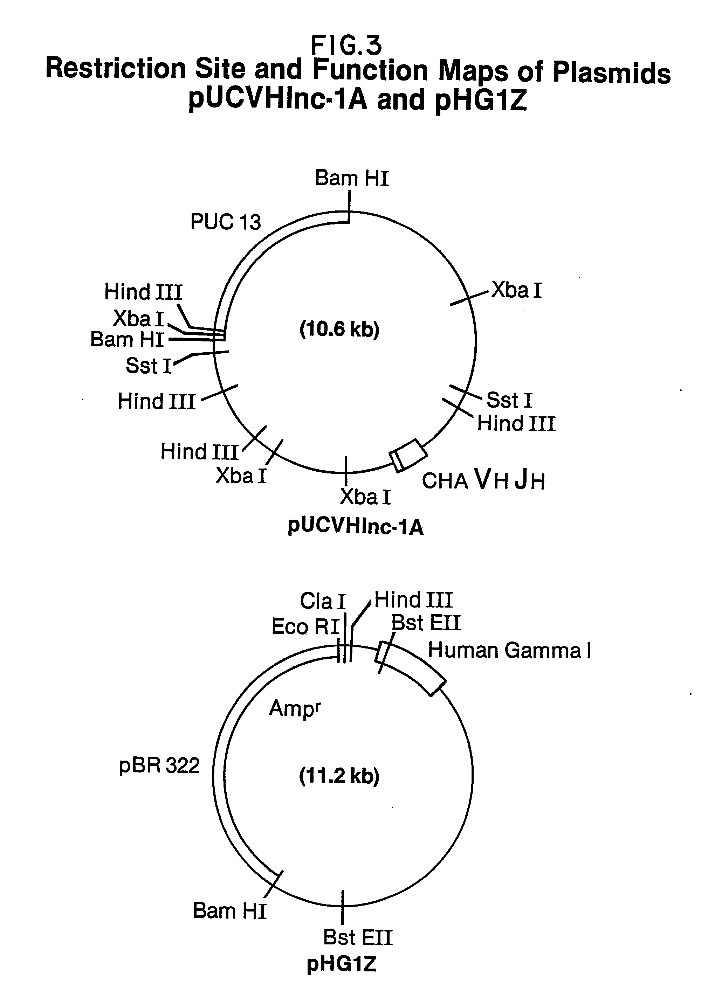

One μg of DNA from the phage φMVHCH-Inc was digested with BamHI and using conditions and buffers supplied by the manufacturer (BRL). DNA (1 μg) from pUC13 (New England Biolabs) was also digested with BamHI. These two DNAs were then ligated together using T4 DNA ligase. A bacterial strain of E. coli JM109 (Stratagene) was transformed with this DNA and then plated on plates containing 2 μg/ml IPTG, 4 μg/ml X-Gal, and 100 μg/ml ampicillin. White colonies were isolated and DNA was analyzed by restriction digestion essentially as described in Maniatis, p. 368-369. The restriction maps confirmed the identify of a plasmid pUCVHInc-1A in which the 8.6 kb BamHI insert contained the CHA255.5 specific Vh gene. A culture of E. coli K-12 HB101/pUCVHInc-1A was deposited with the NRRL on November 14, 1988 and can be obtained under the accession number NRRL B-18433. A restriction site and function map of this plasmid is shown in Figure 3 of the Drawings.

EXAMPLE 8

Plasmid pHG1Z - Construction of a Plasmid Containing Human Constant Region Heavy Chain Gene

Part A: Isolation of Human DNA

Human DNA was isolated as described and detailed in Example 2 Part D above.

Part B: Construction of a Human Plasmid Library

Human DNA of the azg haplotype was digested as 10 μg aliquots using 30 units each of the restriction endonucleases BamHI and HindIII in Reaction Buffer #3 (50 mM Tris-HCl pH 8.0, 10 mM MgCl$_2$, 100 mM NaCl) in a total volume of 200 μl. The digested fragments were concentrated to 20 μl by ethanol precipitation and separated on a 0.6% low gelling temperature agarose (FMC) gel run for 15 hrs at 50 mAmps. DNA fragments in the size range 6-7 kb were excised from the gel. The cloning vector pUC 18 (described by Yanisch-Perron C., et al., Gene 33:103 (1985) and available from New England Biolabs) was also digested with BamHI and HindIII as described above. The human DNA fragments (150 ng) were ligated into the pUC18 vector (50 ng) in a total reaction volume of 400 μl containing 30 mM Tris-HCl (pH 7.6), 10 mM MgCl$_2$, 5 mM dithiothreitol, 1 mM ATP, and 1μl T4 DNA ligase (2 units) for 72 hours at 15° C. Half (100 ng) of the ligated DNA sample was used to transform 500 μl of freshly prepared, competent E. coli JM109 cells (Stratagene) and the resulting transformants were plated onto X-gal, IPTG, AMP plates (4 μg/ml X-gal, 2 μg/ml IPTG, 100 μg/ml Ampicillin).

Part C: Final Construction of Recombinant Plasmid pHGIZ

Ampicillin-resistant pUC18 colonies containing recombinant human DNA were screened by the method described in Example 2E using a human Gamma 2 probe supplied by T. Honjo, University of Osaka, Japan and described by Takahashi, N. et al., Cell, 29:671.679 (1982). A clone containing a 7.5 kb insert corresponding to the human Gamma 1 gene was identified and designated HyHG1. This same 7.5 kb HindIII - BamHI fragment containing the human gamma constant region gene was subsequently re-cloned into pBR322 using the same methodology as described in Example 3A. The pBR322 vector containing the human Gamma 1 gene was designated pHGIZ and was deposited under the Budapest Treaty with the American Type Culture Collection on March 1, 1988 (ATCC Deposit #67638). A restriction site and function map of this plasmid is included in Figure 3 of the Drawings.

## EXAMPLE 7

Plasmid pHGI-CHA: Construction of a Vector Containing Both the CHA 255 $V_H$ Region Gene and Human $C_H$ Region Gene

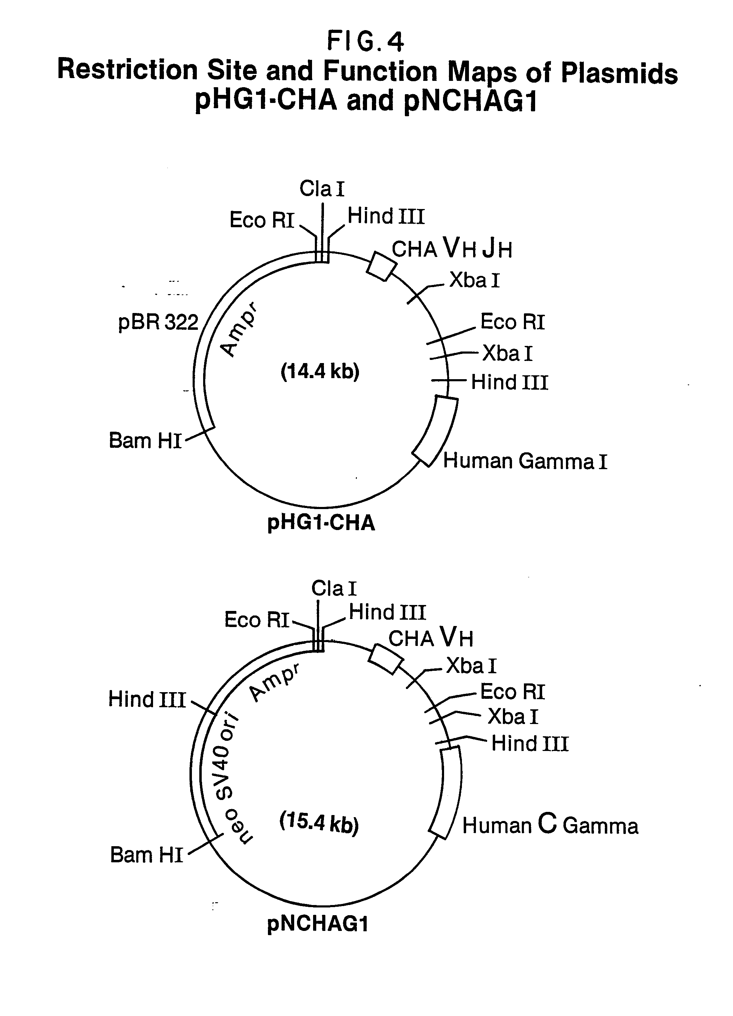

Plasmid DNA from pUCVHInC-Ia was extracted as described in Maniatis, pp. 86-96. The DNA (10 $\mu$g) was then digested with the enzyme HindIII using React buffer #2. The digested DNA was then electrophoresed overnight in a 0.7% agarose gel using Tris Borate Buffer. A 3.4 kb fragment was isolated on DEAE 81 paper. DNA (1 $\mu$g) from plasmid pHGIZ (ATCC Deposit #67638) was also digested with HindIII and then treated with calf intestinal alkaline phosphatase (Boehringer Mannheim) using the protocol supplied with the enzyme Ligations were done in 10 $\mu$l-total volume using 200 ng of phosphatased pHGIZ and 100 ng of the 3.4 kb VH HindIII fragment from pUCVHInc-IA. Ligations were done overnight at 12°C. The ligated DNA was used to transform the bacterial cell line HB101 (BRL) and the transformed cell line was plated on ampicillin plates. Colonies were screened by first tranferring them to a nitrocellulose filter and then hybridizing them with the 3.4 kb VH fragments which had been nick-translated using standard protocols (Grunstein and Hogness, (id.,) 1975 and Rigby et al., id.). Colonies which hybridized to the VH probe were then grown overnight in 5 ml of LB which contained ampicillin (50 $\mu$g/ml) and the DNA was isolated and analyzed by restriction digests. A plasmid containing the CHA255.5 VH specific gene fused to a human Gamma 1 constant region gene was identified and designated pHG1-CHA. This plasmid constituted formation of a chimeric immunoglobulin heavy chain gene wherein the Vh region is derived from a murine hybridoma of known specificity and the constant region is derived from a human source. A restriction site and function map of this plasmid is included in Figure 4 of the Drawings.

## EXAMPLE 8

Plasmid pNCHAGI: Construction of a Mammalian Expression Vector Containing Both the CHA 255 $V_H$ Region Gene and the Human Gamma 1 $C_H$ Region Gene

Part A: Plasmid pSV2neo-Cla

A chimeric immunoglobulin vector capable of being expressed in mammalian cells was constructed next using the chimeric portion of pHG1-CHA and the mammalian expression vector pSV2neo, available from the American Type Culture Collection, (ATCC Designation #37149). pSV2neo DNA (1 $\mu$g) was digested with the EcoRI using 1 unit/$\mu$g of DNA in Reaction Buffer #3. The EcoRI ends were then made blunt by adding 10 $\mu$l of a solution with a concentration of 5 mM each of the 4 deoxyribonucleotides dTTP, dGTP, dCTP and dATP, 2 units of Klenow enzyme and 10X Ligase buffer (0.5 M Tris HCl (pH 7.5); 0.1 M $MgCl_2$; 10 mM diothiothreitol) in a total volume of 50 $\mu$l as described in Maniatis, pp. 113. The reaction was incubated for 30 minutes at room temperature and was followed by a ligation reaction in which phosphorylated ClaI linkers (2 $\mu$g) (New England BioLabs) were ligated with T4 DNA Ligase to 500 ng of EcoRI blunt-ended pSV2neo in order to create a new ClaI site for our future chimeric vector. The ClaI linkers was d(pCATCGATG). Ligation reactions were carried out as described under Example 2E. Following ligation, excess linkers were removed by electrophoresis of the DNA and the linear pSV2neo-Cla fragment onto DEAE 81 paper was isolated using the Example 2E. The isolated DNA was self-ligated using reagents described in Example 4A. HB101 competent cells (BRL) were transformed as in Example 2E and the ampicillin resistant colonies analyzed by restriction enzyme digestion.

The resulting vector, pSV2neo-Cla, was then digested simultaneously with ClaI and BamHI restriction enzymes (1 unit/$\mu$g of DNA). The chimeric vector pHG1-CHA was also digested with these two enzymes. The 5.0 kb pSV2neo-Cla-Bam fragment and the 10.5 kb Cla-Bam pHG1-CHA fragment were isolated on DEAE 81 paper as described in Example 2E. A standard ligation reaction, as described in Example 2, part D, was done using 300 ng of the 10.5 kb fragment insert DNA and 150 ng of the 5.0 kb vector pSV2neo-

Cla. Following transformation of HB101 cells (BRL), a recombinant plasmid, designated as pNCHAG1, was identified by restriction mapping of the plasmid DNA. The restriction site and function map of this plasmid, which is the chimeric expression vector used to produce the CHA chimeric heavy chain polypeptide, is shown in Figure 4 of the Drawings.

## EXAMPLE 9

### DNA Sequencing of Cloned Genes

Sequencing of the cloned CHA variable light and heavy chain genes was accomplished by standard procedures for both double and single stranded templates using the protocols provided by the sequencing kit Sequenase (U.S. Biochemicals, Cleveland, Ohio), and the Bluescript/DNA Sequencing System (Stratagene). From the DNA sequences obtained for the cloned CHA variable light and heavy region genes, the amino acid sequences of the polypeptides encoded by the DNA sequences were deduced by a computer software program, MAPSEQ, commercially available from DNAStar (Madison, Wisconsin).

## EXAMPLE 10

### Transfection of Mammalian Cell Lines with Constants Containing Chimeric Heavy and Light Chain Genes

Part A: Transfection of Chimeric Light Chain Gene With the Chimeric Construct pGCHAK

The light chain immunoglobulin plasmid used for transfection was pGCHAK, as described in the Example 1 above. The pGCHAK plasmid, containing the chimeric variable light ($V_K$) CHA gene fused to the human kappa gene, was first transfected into SP2/0 hybridoma cells (ATCC) by the electroporation techniques referenced above. The SP2/0-Ag14 cells were grown in media (such as HH₂, DMEM, RPMI) containing 5% FCS and maintained in a log phase of growth for the three days preceeding electroporation. The plasmid vector pGCHAK (20 $\mu$g) was linearized using the restriction enzyme PvuI (1 u/$\mu$g) and Reaction Buff #7. At the time of transfection the SP2/0 cells were collected by centrifugation in an IEC clinical centrifuge - (800 rpm, 10', room temperature). Cells were then washed three times in Hanks Buffered Saline Solution (Gibco) with 6 mM dextrose and resuspended at a final concentration of 1.5 x 10⁶ cells/ml. A portion (0.3 ml) of the cells were aliquoted into cuvettes at a density of 0.5 x 10⁶/0.3 ml and the linearized DNA was added. The mixture was maintained on ice for 10 minutes. Electroporation was done using the 0.8 mm gap electrode (P/N 472) and the BTX 100 Transfector (BTX, Inc. San Diego, Ca.). One pulse of 5 milliseconds duration at 250 volts were used. The electroporated cells were then resuspended in media at a density of 2 x 10⁵/ml (in T75 flasks) for 72 hours. (37° C, 5% CO₂ atmosphere). Cells were then plated in the appropriate antibiotic at a density of 5 x 10⁴/ml in 24 well plates; SP2/0 cells containing pGCHAK were plated in HMAX 1.0 Media (50 ng/ml hypoxanthine, 250 ng/ml mycophenolic acid and 50 $\mu$g/ml xanthine, (Sigma)) at 1 $\mu$g/ml. Supernatant (200 $\mu$l) was collected from each well which contained HMAX resistant colonies. This supernatant was then assayed as described below for the presence of a human kappa constant region gene which would indicate expression of the chimeric immunoglobulin genes of pGCHAK.

Part B. Identification of SP2/0 Cells Secreting Chimeric CHA 255.5

Transfected SP2/0 cells expressing the chimeric CHA-human kappa genes were identified by a standard enzyme-linked immunosorbent assay (ELISA), as described by Engvall, E. and Perlmann, P., Immunochemistry 8:871-874 (1971), for human kappa. The purpose of this assay was to identify those cells secreting the chimeric kappa chain polypeptide coded for by the pGCHAK plasmid vector which was constructed from murine variable regions isolated from the murine hybridoma CHA 255.5 and fused to the human kappa gene. A 5 $\mu$g/ml solution of goat anti-human kappa chain (Tago #4106) in 10 mM sodium phosphate pH 7.8 was prepared. Each well of a 96 well plate was coated with 50 $\mu$l of this solution. The plates were then incubated overnight at 37° C. Plates were then rinsed thoroughly in $H_2O$ and PBS + 0.1% Tween (w/v). Fifty $\mu$l of the supernatant fractions were added to each well, and incubated for 2 hours at room temperature. Plates were again rinsed as detailed above. A goat anti-human kappa chain alkaline phosphatase conjugate (Tago #2496) was diluted 1:1000 in the same medium as the supernatant material. 100 $\mu$l were added per well and allowed to incubate for 1 hour at room termperature. Plates were rinsed as above. The alkaline phosphatase substrate was prepared as per package instruction, 1 tablet per 3 ml of distilled $H_2O$ and 150 $\mu$l of this substrate was added to each well and allowed to incubate 30 minutes at 37° C. The reaction was quenched with 50 $\mu$l of 500 mM EDTA and then the absorbance was read at 405 nm. Those supernatants showing the highest levels of kappa expression were identified and the cells from the corresponding wells were pooled and expanded for introduction of the chimeric construct pNCHAG1.

Part C: Transfection of Chimeric Kappa Producing Cells with the Heavy Chain Chimeric Construct pNCHAG1

The heavy chain immunoglobulin plasmids used for transfection into SP2/0 cells was pNCHAG1, derived from constructs as detailed in Example 8. The populations of cells expressing the chimeric CHA-human kappa genes which were pooled were next electroporated with the plasmid constructs containing the chimeric CEM heavy chain genes. As for the kappa gene electroporation the SP2/0 chimeric kappa producing cells (SP2/0-K) were maintained at log phase of growth for the three days preceeding the electroporation. Twenty micrograms of the plasmid DNA pNCHAG1 was linearized with the enzyme PvuI in React buffer #7. Cells were collected, washed and resuspended at a density of 1.5 x $10^7$ cells/ml as detailed in Example 2A. The DNA was added and the mixture held on ice for 10 minutes preceeding the electroporation. Conditions used were 1 pulse at 5 m seconds, 250 volts. Cells were plated at 2.5 x $10^5$/ml in mammalian tissue culture media, such as DMEM, RPMI or HH2 (the later is from Hybritech Incorporated, San Diego, CA.), 5% FCS plus HMAX 1.0 for 72 hours at 37° C, 5% $CO_2$ atmosphere. Following this cells were plated at 5 x $10^4$/ml in 24 well plates in medium containing HMAX 1.0 and G418 antibiotic (Geneticin, Gibco-BRL,) at an active concentration of 500 $\mu$g/ml. Selection was maintained for 14 days at which time those wells with HMAX/G418 resistant colonies were identified for further analysis.

EXAMPLE 11

Identification and Analysis of Chimeric Antibody SP2/0 Cells Secreting CEM-Chimeric Antibody

Part A: Antigen Binding

Screening assays were performed to identify transfected SP2/0 cells which expressed both the chimeric CHA light and heavy chain immunoglobulin genes producing antibody which binds benzyl EDTA hapten. The assay procedures used for the detection of antibodies directed against the metal complex antigen were standard solid phase radioimmunoassays, essentially as described by Wang, R., et al., Immunol. Methods, 18:157-164 (1977). The assay was carried out in the following manner. BSA was derivatized with benzyl-EDTA according to the procedure of Example 1. Each molecule of BSA contained 5-10 chelate molecules. The chelates were loaded with indium by adding 105% molar excess of $InCl_3$ over hapten to the BSA-chelate solution and allowed to incubate overnight in 0.13 M citrate pH 6. The solution was brought to volume with 10 mM phosphate pH 7.2. This BSA-EDTA-In solution was brought to a concentration of 10 $\mu$g/ml and 50 $\mu$l was added per well of a 96 well microtiter plate. Plates were dried overnight at 37° C. Prior to assay, the plates were washed with PBS and 0.1% Tween 20 and deionized water. Each well received 50 $\mu$l of supernatant which was incubated for two hours at room temperature. Wells were washed with PBS + 0.1% Tween and deionized water again. Goat anti-human kappa conjugated to alkaline phosphatase (Tago #2496) was diluted 1:500 and 100 $\mu$l was added to each well. This was incubated one hour at room temperature. Wells were washed as they have been previously. Each well received 150 $\mu$l substrate. The enzymatic reaction was incubated 60 minutes at 37°C on a rotator. The reaction was quenched with 50 $\mu$l of 500 mM EDTA pH 8. Absorbance at 405 nM was determined using a BIO-TEK Elisa Reader.

Part B: Antibody Affinity

Affinity assays were performed to determine the Ka of the present chimeric antibody CHA255.5 for the indium (III) complex of EOTUBE. EOTUBE is a derivative of p-(aminobenzyl) EDTA and is described in L.D. Andersen, J.M. Frincke, and D.L. Meyer, U.S. Patent No. 151855, filed 2-3-88, (European Patent Application No. 89301012.4) herein incorporated by reference.

The synthesis of EOTUBE and its use in a standard Scatchard Analysis are set forth below.

1. Synthesis of EOTUBE

Specifically, EOTUBE is EDTA substituted at one of the internal ethylene carbons (S stereochemistry at that substituted carbon) through the benzylic carbon of an N-(2-hydroxyethyl)-N'-(p-benzyl) thiourea moiety. The synthesis of EOTUBE was performed excluding metal ions as much as possible. (For example, all glassware was washed with 6 M HCl, and only deionized water was used).

(S)-p-nitrobenzyl EDTA was prepared and converted to (S)-4-isothiocyanatobenzyl EDTA (hereinafter abbreviated as ITCBE) as described in U.S. Patent No. 4,622,420, and Meares, C.F., Anal. Biochem., 142 68-75 (1984). The lyophilized ITCBE was resuspended in 0.3 M HCl (Ultrex, J.T. Baker, Phillipsburg, N.J.) to a final concentration of roughly 50 mM. This solution was stored at -70°C. Unless indicated otherwise, all reactions were done in aqueous solution as 40°C.

2.5 ml of 20 mM ITCBE was added to 1.35 ml of 200 mM ethanolamine and the pH adjusted to 11.0 with 11 N NaOH. The volume was adjusted to 5 ml with water and the mixture allowed to react for 15 minutes, at which time it was checked by HPLC analysis. All of the ITCBE was converted to EOTUBE, with a retention time of 3.6 minutes on a HPLC (C18 column, eluted with a linear gradient of aqueous buffer of 50 mM triethylammonium acetate to neat methanol on a Hewlett-Packard 1090 instrument).

The product was purified by anion exchange chromatography on an 11 ml column of DEAE Sephadex A-25, eluted with a 110 ml gradient of 0.1 to 1 M ammonium formate, pH 6. (The column was monitered at 280 nm.) Fractions containing the product were pooled and lyophilized. The product had an absorbance maximum at 246 nm with an extinction coefficient of 18000.

The structure was purified by carbon 13 NMR spectroscopy. (A Varian Instruments Model XL-300 300 Mhz instrument was used. The spectrum was run deuteriated water.) The peak corresponding to the carbon in the isothiocyanate moiety in ITCBE was at 139 ppm, and was replaced by a peak at 182 ppm in EOTUBE. This peak corresponds to the carbon in the thiourea linkage. The aromatic region (128-138 ppm) of the spectrum of ITCBE shows four peaks, while that of EOTUBE shows three. In the aliphatic region. there are 5 peaks in common for ITCBE and EOTUBE, and an additional two peaks at 64 and 49 ppm in the EOTUBE spectrum. The latter peaks correspond to the carbons adjacent to the hydroxyl and thiourea moieties, respectively.

Procedure

I. RADIOLABEL OF CHELATE

    A. Label $9.9540 \times 10^{-7}$ mmoles of EOTUBE with 600 $\mu$Ci [111]In.
      1. Without introducing any other metal add the following to a metal-free tube:
a. 63 $\mu$l 0.0158 mM metal-free EOTUBE
b. 63 $\mu$l 0.26 M metal-free ammonium citrate pH = 6.0
c. 600 $\mu$Ci $^{111}$In ($1.30 \times 10^{-9}$ mmoles)
      2. Incubate overnight at room temperature.
    B. Load step A. with enough cold indium to give a 1.05 molar ratio of indium (both $^{111}$In and ground state

In) over EOTUBE.

 1. To the tube from above (I.A.2 ) add 10.2 $\mu$l 0.012 mM $^{ground\ state}$ InCl$_3$ (1.044 x10$^{-6}$ mmoles).

 2. Incubate 4 hours at room temperature.

 C. Perform Thin-Layer Chromatography analysis to test incorporation of indium.

 1. Spot 0.5 $\mu$l of the labeled sample in duplicate lanes 1 cm from the bottom of a silica gel plate 10.5 inches long marked off in 0.25 inch strips to make lanes.

 2. Allow the sample spots to dry.

 3. Place plate in a TLC tank with a 10% ammonium acetate:methanol (1:1) solvent system.

 4. Develop plate to the 9 cm mark.

 5. Remove plate and allow to dry.

 6. Cut each lane of the plate into 3 sections.

a. The bottom to the 2 cm mark is the origin section.

b. 2 cm-3.5 cm is the middle section.

c. 3.5 cm-top is the tail section.

 7. Count the cpms of each section for both lanes.

 8. The percent at the tail for each lane is equal to the percent indium incorporated into the chelate.

 D. If indium incorporated greater than 90% dilute EOTUBE to 4.40 pg/$\mu$l in PBS pH = 7.5 for use in assay.


II. COLD COMPETITOR PREPARATION

 A. Cold indium load 5.530 x 10$^{-4}$ mmoles EOTUBE with a molar ratio of 1.02 X InCl$_3$ over EOTUBE.

 1. Without introducing any contaminating metal add the following to a metal-free tube:

a. 70 $\mu$l 7.9 mM metal-free EOTUBE (5.530 x 10$^{-4}$ mmoles).

b. 70 $\mu$l 0.26 M metal-free ammonium citrate pH = 6.0

c. 55.3 $\mu$l 10.2 mM $^{ground\ state}$InCl$_3$ (5.641 x 10$^{-4}$ mmoles).

 2. Incubate 4 hours at room temperature.

 B. Dilute to get 1 ml of 0.40 ng/$\mu$l and 1 ml of 0.36 ng/$\mu$l in PBS pH = 7.5 for use in part IV.


III. ANTIBODY SENSITIVITY CURVE

 A. To a 96 well microtiter plate add the following in triplicate:

 1. 25 $\mu$l EOTUBE-$^{111}$In-$^{ground\ state}$In at 4.4 pg/$\mu$l.

 2. 25 $\mu$l antibody dilution (CHA255 or derivative)- either 20 $\mu$g/ml, 10, 5, 2.5, 1.25, 0.623, 0.313, 0.156, 0.080, 0.040, 0.020, or 0 $\mu$g/ml.

 3. 50 $\mu$l RPMI with 10% horse serum.

 4. 20 $\mu$l sheep anti-mouse IgG coupled to sepharose beads (March, S.C., Parikh, I. and Cuatrecacas, P. Analyt. Biochem. 60, 149 (1974) diluted to a concentration so that 20 $\mu$l will bind 0.5 $\mu$g Ab.

 B. Incubate overnight on a rotator at room temperature.

 C. Aspirate contents of wells onto glass-fiber filter paper.

 D. Cut out and count filter discs.

 E. Graph antibody dilution # vs. fraction bound.

 F. Determine what point (dilution #) equals 90% of maximum bound.

 G. Use the determined dilution from above for the scatchard assay.


IV. SCATCHARD ASSAY

 A. To a 96 well microtiter plate add the following in triplicate:

 1. 25 $\mu$l EOTUBE-$^{111}$In-$^{ground\ state}$In at 4.40 pg/$\mu$l.

 2. 25 $\mu$l EOTUBE-$^{ground\ state}$In at serial dilutions of the 0.40 ng/$\mu$l and 0.36 ng/$\mu$l made in part IIB. Eleven dilutions each plus a zero giving 24 points with a range of 10 ng/well down to 4.40 pg/well plus a zero. Dilute with RPMI containing 10% horse serum.

 3. 25 $\mu$l RPMI containing 10% horse serum.

 4. 25 $\mu$l antibody of interest. (concentration determined by sensitivity assay).

 5. 20 $\mu$l sheep anti-mouse IgG coupled to sepharose beads (same as used in sensitivity curve assay).

 B. Incubate overnight on a rotator at room temperature.

 C. Aspirate contents of wells onto glass-fiber filter paper.

D. Cut out and count filter paper discs.

E. Plot Molar Bound vs. Bound/Free.

F. Take linear regression of the part of the curve that is a straight line. The negative of the slope is the Ka.

## EXAMPLE 12

### Construction of an SV40 Enhancerless Cloning System

#### A. Construction of Plasmid pSV2gpt(E-)

About 20 µl (10 µg) of plasmid pSV2gpt-Cla (constructed in Example 4A3) were mixed with 21 µl water, 5 µl React buffer #6, 2 µl restriction enzyme PvuII and 2 µl restriction enzyme SphII, then incubated for 2 hours at 37°C. The reaction mixture was electrophoresed through a 0.5% TBE gel and the PvuII/SphII-digested vector fragment was purified from DEAE 81 paper in substantial accordance with the teaching of Example 2E. To fill in the 3' protruding ends of these restriction sites, about 20 ml of the PvuII/SphII digested pSV2gpt-Cla vector were mixed with 3 µl 10 x T4 polymerase buffer (700 mM Tris, pH 7.4; 100 mM MgCl₂: 50 mM DTT), 3 µl of dNTP mix (0.5 mM each of dATP, dTTP, dCTP, and dGTP, pH 7.0), 3 µl of water and 1 µl (5U) of T4 DNA polymerase (BRL). The mixture was incubated at 37°C for 15 minutes, then heated to 70°C for 10 minutes. After a phenol extraction and ethanol precipitation, the DNA was resuspended in 5 µl of TE buffer. About 1 µl (about 500 µg) of this DNA fragment was mixed with 10 µl of water, 5 µl of 5x ligation buffer, 2 µl of 100 mM ATP and 2 µl of T4 ligase. After an incubation of 2 hours at room temperature, the ligation mixture was used to transform into E. coli HB101 cells in substantial accordance with the teaching of Example 2E. Plasmid DNA was isolated from the transformants and those plasmids which displayed the proper approximately 0.2 kb deletion of the SV40 enhancer region were designated plasmid pSV2gpt(E-).

#### B. Construction of Plasmid pSV2neo(E-)

An SV40 enhancerless pSV2neo plasmid was also constructed. About 20 µl (10 µg) of plasmid pSV2neo-Cla (constructed in Example 8A) were digested with restriction enzymes BamHI and HindIII in a reaction mixture using React buffer #3, substantially as described above. The approximately 2.3 kb HindIII/BamHI neomycin resistance-confering gene containing fragment was isolated and purified from DEAE 81 paper following electroelution. In a similar manner, plasmid pSV2gpt(E-) was also digested with restriction enzymes BamHI and HindIII and the large vector fragment was purified after electrophoresis. The about 2.3 kb HindIII/BamHI neo containing restriction fragment plasmid pSV2neo-Cla was then ligated into the HindIII/BamHI-digested vector fragment of plasmid pSV2gpt(E-) in substantial accordance with the teaching of the previous paragraph. After transformation into E. coli HB101 and isolation of plasmid DNA, those plasmids in which the pSV2gpt(E-) vector backbone was ligated with the about 2.3 kb BamHI/HindIII neo fragment of plasmid pSV2neo-Cla were designated plasmid pSV2neo(E-).

#### C. Construction of Plasmids pGCHAK(E-) and pGCHAG(E-)

##### 1. Plasmid pGCHAK(E-)

About 10 µg (100 µl) of plasmid pSV2gpt(E-) DNA were mixed with 4 µl of water, 12 µl of React buffer #1, 2 µl of restriction enzyme ClaI and 2 µl restriction enzyme BamHI, then incubated overnight at 37°C. After electrophoresis onto DEAE 81 paper, the large vector fragment was purified and resuspended in 10 µl TE. About 20 µl (5 µg) of plasmid pGCHAK (constructed in Example 4B) were mixed with 23 µl water, 5 µl of React buffer #1 and 2 µl restriction enzyme ClaI. After 5 hours at 37°C, the reaction was extracted with phenol/choroform and ethanol precipitated. This digested DNA was then resuspended in 25 µl of water and mixed with 3 µl of React buffer #3 and 2 µl restriction enzyme BamHI. Following a 2 hour incubation at 37° C, the digested DNA was electrophoresed and the approximately 9.0 kb murine variable, human constant kappa-encoding ClaI/BamHI restriction fragment was purified from DEAE 81 paper. This approximately 9.0 kb fragment was then ligated into the ClaI/BamHI-digested plasmid pSV2gpt(E-) and transformed into E. coli cells as previously described. After plasmid isolation, those plasmids which comprise the correct restriction maps were designated plasmid pGCHAK(E-).

2. Plasmid pGCHAGI(E-)

In a similar fashion, about 40 µl (5 µg) of plasmid pNCHAG1 (constructed in Example 8A) were mixed with 3 µl of water, 5 µl of React Buffer #1 and 2 µl restriction enzyme ClaI. After 5 hours at 37° C, the reaction was extracted with phenol/chloroform and ethanol precipitated. This digested DNA was resuspended in 26 µl of water, 3 µl of React buffer #3, and 1 µl of restriction enzyme BamHI. After 2 minutes at 37° C, 15 µl of the reaction mixture was removed and mixed with 1 µl of 250 µM EDTA. After 5 minutes at 37° C, the remaining reaction mixture was also removed and mixed with 1 µl of 250 µl of 250 µM EDTA. This partial BamHI digestion yielded all possible ClaI/BamHI restriction fragments. After electrophoresis on a 0.5% TBE gel the approximately 12.7 kb ClaI/BamHI (partial) restriction was then ligated into the ClaI/BamHI-digested plasmid pSV2gpt(E-), then transformed into E. coli. These plasmids are designated plasmid pGCHAGI(E-).

D. Construction of Plasmids pNCHAK(E-) and pNCHAGI(E-)

1. Plasmid pNCHAK(E-)

About 10 µg (100 µl) of plasmid pSV2neo(E-) DNA were digested with restriction enzymes ClaI and BamHI, then the vector fragments were isolated and purified in substantial accordance with the teaching of Example 12C. The approximately 9.0 kb ClaI/BamHI restriction fragment of plasmid pGCHAK (isolated in Example 12), which comprises the murine lambda variable, human constant region encoding genes, was then ligated into the ClaI/BamHI digested fragment of plasmid pSV2neo(E-) to form plasmid pNCHAK(E-), all in substantial accordance with the teaching of Example 12C.

2. Plasmid pNCHAGI(E-)

Also in accordance with the teaching of Example 12C, plasmid pNCHAG(E-) is constructed by ligating the approximately 12.7 kb ClaI/BamHI (partial) restriction fragment of plasmid pNCHAGI into the ClaI/BamHI digested fragment of plasmid pSV2neo(E-). Plasmid pNCHAG(E-) therefore comprises the murine variable, human constant gammaI encoding genes on an SV40 enhancerless expression vector.

EXAMPLE 13

Transfection of Chimeric Light and Heavy Chain Genes with SV40 Enhancerless Vectors

The two SV40 enhancerless expression vectors for the light chains (pGCHAK(E-) and pNCHAK(E-)) and also the heavy chain expression vectors with the SV40 enhancer (pGCHAGI and pNCHAGI) are transfected into SP2/0 hybridoma cells by electroporation substantially as described in Example 10. Each plasmid can be singly transfected, or co-transfected, into the cells, although the best results occur when a vector comprising the kappa chain and the gpt marker are first transfected into the cell, followed by a second transfection of a vector comprising the gamma chain and the neo marker. Following growth on any conventional mammalian cell culture media, including serum free media and selection with the appropriate antibiotic (HMAX for cells containing a gpt vector; G418 for cells containing a neo vector), the cells are assayed for whole antibody secretion in substantial accordance with the teaching of Examples 10 and 11. The results will demonstrate that expression of chimeric antibodies or antibody light chains is increased in those cells which have been transfected with SV40 enhancerless light chain vectors.

## EXAMPLE 14

## Construction of a High Level Expression System for Expression of Heterologous Immunoglobulin Chains

### A. Construction of Plasmid p19HANCH

Intermediate plasmid p19HANCH was first constructed by preparing an oligonucleotide polylinker with the DNA sequence comprising:

```
5'-AATTCAGATCTGGTGACCCGCGGTCGACGGGCTGCAGAATATTGCGGCCGCCATGGATCCA-3'
     |||||||||||||||||||||||||||||||||||||||||||||||||||||||||||
  3'-GTCTAGACCACTGGGCGCCAGCTGCCCGTCGTCTTATAACGCCGGCGGTACCTAGGTTCGA-5'
```

The linker depicted above was synthesized from single-stranded deoxyoligonucleotides by procedures well known in the art. The single-stranded deoxyoligonucleotides can be synthesized with commercially available instruments, such as the Biosearch Model 8700 DNA Synthesizer marketed by Biosearch Incorporated, San Rafeal, CA, which utilizes phosphoramidite chemistry. Other procedures for synthesizing DNA are also known in the art. The conventional modified phosphotriester method of synthesizing single DNA is described in Itakura et al. 1977, Science 198:1056 and in Crea et al., 1978, Proc. Nat. Acad. Sci. USA 75:5765. In addition, an especially preferred method of synthesizing DNA is disclosed in Hsuing et al., 1983, Nucleic Acid Research 11:3227 and Narang et al., 1980, Methods in Enzymology 68:90.

About 2.5 μg of each of the two oligonucleotide strands were mixed with 50 μl 2x Annealing Buffer (0.2 M NaCl, 20 mM Tris-HCl (pH 7.8) and 2 mM EDTA) and 50 μl of water. The reaction mixture was heated to 70°C for 5 minutes then allowed to slowly cool to room temperature so that the two single strands would anneal into one linker segment. Approximately 1 μg of plasmid pUC19 (New England Biolabs) was digested with restriction enzymes EcoRI and HindIII in substantial accordance with the above teachings. After purification of the about 2.6 kb vector fragment, the synthesized polylinker was ligated into the EcoRI/HindIII digested plasmid pUC19. After transformation into E. coli and re-isolation of plasmid DNA, those plasmids which demonstrated the HindIII SspI, PstI. SstII and EcoRI sites within the linker region were designated plasmid p19HAN.

Plasmid S(-)CHAVL was constructed by first digesting the Bluescript vector M13(-)SK (Stratagene) with restriction enzymes BamHI and SstI then isolating the large vector fragment in substantial accordance with earlier teaching. Plasmid pMLCH-1 was next digested with restriction enzymes BamHI and SstI, and the approximately 1100 bp fragment which comprises the gene encoding the murine CHA variable region was isolated. Plasmid pMLCH-1 can be conventionally isolated from E. coli K12 HB101/pMLCH-1, a strain deposited and made part of the permanent stock culture of the Northern Regional Research Laboratory, 1815 North University Street, Peoria, IL 61604. E. coli K12 HB101/pMLCH-1 is available under the accession number NRRL B-18432. The approximately 1100 bp BamHI/SstI restriction fragment of plasmid pMLCH-1 was then ligated into the BamHI/SstI digested vector M13(-)SK and the plasmid was transformed into E. coli in substantial accordance with prior examples. Following re-isolation and restriction mapping, those plasmids which contained the proper approximately 1100 bp BamHI/SstI fragment were designated plasmid S(-)CHAVL.

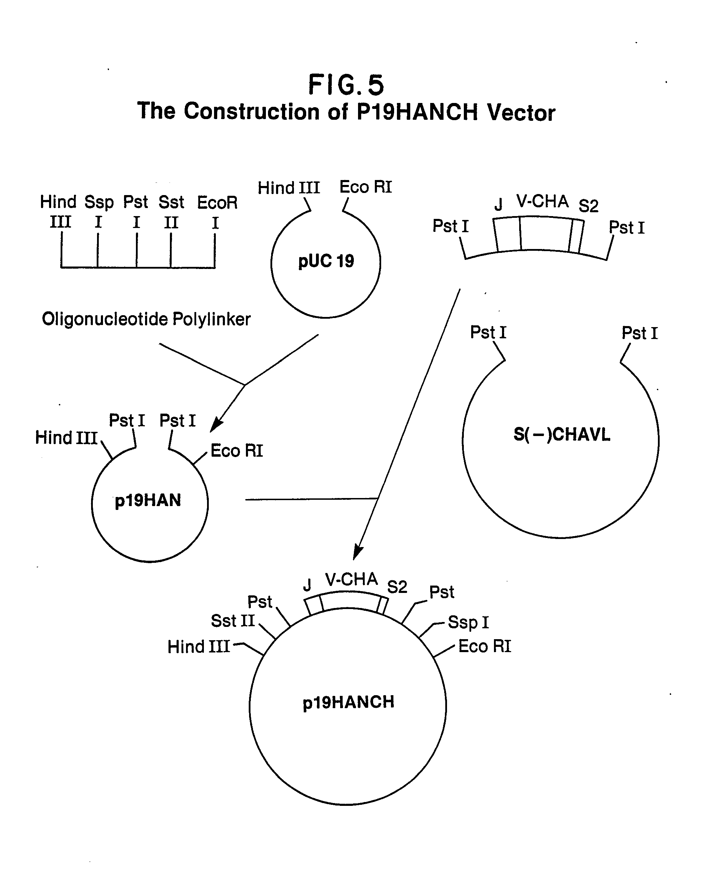

About 1 μg of plasmid S(-)CHAVL was next digested with restriction enzyme PstI in substantial accordance with prior teachings. An approximately 900 bp PstI restriction fragment which comprises the gene encoding the murine CHA variable region was isolated and ligated into plasmid p19HAN, which had first been cut in the polylinker region by restriction enzyme PstI. Following transformation, re-isolation and restriction mapping, those plasmids which comprise the J region of the CHA gene closest to the HindIII site of the polylinker are designated plasmid p19HANCH. The restriction site and function map of plasmids p19HAN and p19HANCH are depicted in Figure 5 of the Drawings.

## B. Construction of Expression Vectors pGCHAK-2 and pGCHAK-3

About 10 μg of plasmid pMLCE-10 (deposited on March 4, 1988 and available from the ATCC under number ATCC 67639) which comprises the gene which encodes for a murine variable kappa chain which reacts with carcinoembryonic antigen, were digested with restriction enzyme HindIII and the approximately 3.8 kb HindIII restriction fragment containing the entire murine variable kappa region and CEM promoter was isolated. About 1 μg of plasmid pHKF-1 (available from the ATCC under the accesion ATCC 67637) was also digested with restriction enzyme HindIII and the largest vector fragment was isolated. The approximately 3.8 kb HindIII fragment of plasmid pMLCE-10 was ligated into the HindIII - digested vector fragment of plasmid pHFK-1 to form plasmid pHKCE-10. Plasmid pHKCE-10 was then digested with restriction enzymes ClaI and BamHI and the approximately 9.0 kb restriction fragment which comprises the kappa promoter, the murine kappa variable region and the human constant region encoding genes were isolated. This approximately 9.0 kb ClaI/BamHI fragment was ligated into ClaI/BamHI digested plasmid pSV2gpt-Cla (constructed in Example 4A3) to form plasmid pGCEMK.

About 5 μg of plasmid pGCEMK was digested with restriction enzymes ClaI and SspI in substantial accordance with earlier teachings, then the approximately 2.2 kb ClaI/SspI restriction fragment, which comprise the CEM kappa promoter, was purified.

In a separate reaction, plasmid pGCEMK was digested with restriction enzymes ClaI and SstII, and the approximately 9.4 kb vector fragment was isolated. Furthermore, plasmid p19HANCH was digested with restriction enzymes SspI and SstII the approximately 931 base pair restriction fragment which comprises the gene encoding the murine kappa CHA variable region was isolated. In a three part ligation reaction, the approximately 9.4 kb ClaI/SstII vector fragment of plasmid pGCEMK, the approximately 2.2 kb ClaI/SstI CEM-promoter containing restriction fragment of plasmid pGCEMK and the approximately 931 base pair SspI/SstII restriction fragment of plasmid p19HANCH which contains the gene encoding the murine kappa CHA variable region were all ligated together and transformed into E. coli in substantial accordance with prior examples. After plasmid isolation and restriction mapping, those plasmids which display the proper restriction fragment sizes are designated plasmid pGCHAK-2.

## pGCHAK-3

### 1) Construction of Plasmid pHKCE-10 Including CEM 231.6.7 V$_L$ Kappa Gene and Human C Kappa Gene

A3.8 kb HindII fragment from pMLCE-10 containing the entire CEM 231.6.7 variable kappa region was further subcloned into the HindIII site of plasmid pHKF-1 using the above methods to form a chimeric plasmid having the murine CEM 231.6.7 variable light region fused to a human constant kappa region gene. One μg of pMLCE-10 DNA was digested with HindIII at 1 unit/μg using React Buffer #2 (Gibco-BRL, Gaithersburg, Maryland). One μg of pHKF1 was digested in a similar manner. The pMLCE-10 digested DNA was electrophoresed as above and the 3.8 kb HindIII fragment was isolated onto DEAE 81 paper and eluted in 5 μl of TE. One μg (2 μul) of HindIII digested pMLCE-10 were ligated to 600 ng of HindIII digested pHKF-1 in the presence of 10x Ligation Buffer, 10 mM ATP and 2 units of T4 DNA ligase of total volume of 10 μl. Ligation was done at 12°C overnight and again HB101 competent cells from BRL were used in the plasmid transformation, as described above. The plasmid designated as pHKCE-10 was identified by restriction mapping of the plasmid DNA.

### 2) Construction of Plasmids pGCEMK(E-)

About 10 μg (100 μl) of plasmid pSV2gpt(E-) DNA were mixed with 4 μl of water, 12 μl of Gibco Reaction Buffer #1, 2 μl of restriction enzyme ClaI and 2 μl restriction enzyme BamHI, then incubated overnight at 37°C. After electrophoresis onto DEAE 81 paper, the large vector fragment was purified and resuspended in 10 μl TE. About 20 μl (5 μg) of plasmid pHKCE-10 were mixed with 23 μl water, 5 μl of Gibco Reaction Buffer #1 and 2 μl restriction enzyme ClaI. After 5 hours at 37°C, the reaction was extracted with phenol/chloroform and ethanol precipitated. This digested DNA was then resuspended in 25 µl of water and mixed with 3 µl of Gibco Reaction Buffer #3 and 2 µl restriction enzyme BamHI. Following a 2 hour incubation at 37°C, the digested DNA was electrophoresed and the approximately 9.0 kb murine variable human constant kappa-encoding ClaI/BamHI restriction fragment was purified from DEAE 81 paper. This approximately 9.0 kb fragment was then ligated into the ClaI/BamHI-digested plasmid pSV2gpt(E-) and transformed into E. coli cells as previously described. After plasmid isolation, those plasmids which comprise the correct restriction maps were designated plasmid pGCEMK(E-).

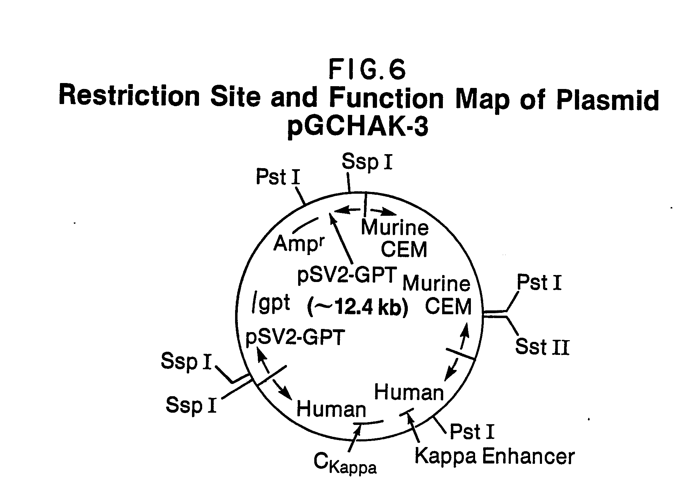

3) In an analogous manner, plasmid pGCEMK(E-) (constructed in Example 12C) was digested with restriction enzymes ClaI and SstII and the approximately 9.2 kb restricion fragment (SV40 enhancerless) was isolated. This fragment was then ligated to the approximately 2.2 kb ClaI/SspI CEM kappa promoter-containing restriction fragment of plasmid pGCEMK and the approximately 931 base pair SspI/SstII restriction fragment of plasmid p19HANCH to form plasmid pGCHAK-3. Plasmid pGCHAK-3 differs from plasmid pGCHAK-2 only in the fact that plasmid pGCHAK-3 lacks the SV40 enhancer. A restriction site and function map for plasmid pGCHAK-3 is included in the Drawing as Figure 6.

## EXAMPLE 15

### Expression of the Heterologous CHA Antibody using the CEM Promoter

### Part A.

The various CHA constructs were transfected into SP2/0 cells in substantial accordance with the teaching of Example 3 and antibody specificity and affinity are measured in substantial accordance with the teaching of Example 4. Initial transfection with plasmid pGCHAK with selection using HMAX followed by a second transfection with plasmid pNCHAGI with selection on both HMAX and G418 yielded low levels of chimeric CHA antibody secretion into the supernatent. Higher levels of antibody production were noted by first transfecting with plasmid pGCHAK-2 followed by transfection with plasmid pNCHAG1. This data demonstrates that the promoter isolated from plasmid pGCEMK is useful to drive high level expression of a heterologous immunoglobulin sequence.

### Part B.

Furthermore, transfection with pGCHAK-3 will demonstrate a 10-fold higher level of kappa chain expression than did the transfection with pGCHAK and pNCHAK. Therefore, the CEM kappa promoter from plasmid pGCEMK can better drive the expression of a heterologous immunoglobulin sequence than the initial expression vector which lacks the SV40 enhancer.

## Claims

1. A recombinant DNA compound comprising a first DNA sequence which encodes for the chelate-specific light chain variable region of a chimeric monoclonal antibody, the first DNA sequence coding for an amino acid sequence comprising:
Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly.

2. The recombinant DNA compound according to Claim 1 wherein the first DNA strand coding sequence comprises:
CAG GCT GTT GTG ACT CAG GAA TCT GCA CTC ACC ACA TCA CCT GGT GAA ACA GTC ACA CTC ACT TGT CGC TCA AGT ACT GGG GCT GTT ACA ACT AGT AAC TAT GCC AAC TGG GTC CAA GAA AAA CCA GAT CAT TTA TTC ACT GGT CTA ATA GGT GGT ACC AAT AAC CGG GCT CCA GGT GTT CCT GCC AGA TTC TCA GGC TCC CTG ATT GGA GAC AAG GCT GCC CTC ACC ATC ACA GGG GCA CAG ACT GAA GAT GAG GCA AGA TAT TTC TGT GCT CTA TGG TAC AGC AAC CTC TGG GTA TTC GGT GGA GGA ACC AAA CTG ACT GTC CTA GGG.

3. The recombinant DNA compound according to Claim 1 or 2 wherein the first DNA coding sequence further comprises a DNA sequence coding for a eukaryotic signal peptide.

4. The recombinant DNA compound according to Claim 1, 2 or 3 wherein the DNA construct further comprises a second DNA strand sequence which codes for the light chain constant region of the chimeric monoclonal antibody.

5. The recombinant DNA compound according to Claim 3 wherein the DNA strand sequence coding for the signal peptide comprises:
ATG GCC TGG ATT TCA CTT ATA CTC TCT CTC CTG GCT CTC AGC TCA GGT GCC ATT TCC.

6. A recombinant DNA compound comprising a first DNA sequence which encodes for the chelate-specific heavy chain variable region of a chimeric monoclonal antibody, the first DNA sequence coding for an amino acid sequence comprising:
Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Ley Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

7. The recombinant DNA compound according to Claim 6 wherein the first DNA strand coding sequence comprises:
GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC.

8. The recombinant DNA compound according to Claim 6 or 7 wherein the first DNA coding sequence further comprises a DNA sequence for a eukaryotic signal peptide.

9. The recombinant DNA compound according to Claim 6, 7 or 8 wherein the DNA construct further comprises a second DNA strand sequence which codes for the heavy chain constant region of the chimeric monoclonal antibody.

10. The recombinant DNA compound according to Claim 8 wherein the DNA strand sequence coding for the signal peptide comprises:
ATG AGC TTT GGG CTG AGC TTG ATT TTC CTT GTC CTA ATT TTA AAA GGT GTC CAG TGT.

11. The recombinant DNA compound according to any of Claims 1 to 10 wherein the first DNA strand coding sequence is derived from a murine hybridoma.

12. The recombinant DNA compound according to Claim 11 wherein the murine hybridoma is CHA 255.5.

13. The recombinant DNA compound according to Claim 4 or 8 wherein the second DNA strand coding sequence is derived from a human lymphocyte.

14. The recombinant DNA compound of Claim 1 or 2 that is plasmid pMLCH-1 as shown in Figure 1 or plasmid pMLCH1dB as shown in Figure 1.

15. The recombinant DNA compound of Claim 6 or 7 that is plasmid pUCVHlnc-1A as shown in Figure 3.

16. The recombinant DNA compound of Claim 4 that is plasmid pGCHAK as shown in Figure 1.

17. The recombinant DNA compound of Claim 8 that is plasmid pHG1-CHA as shown in Figure 4 or plasmid pNCHAG1 as shown in Figure 4.

18. A eukaryotic host cell capable of expression of a chimeric monoclonal antibody comprising the recombinant DNA compound of any of Claims 1 to 5 encoding for the light chain of the chimeric antibody, and transcriptional and translational DNA sequences positioned in relation to the light chain-encoding DNA sequence to direct expression of the light chain.

19. The eukaryotic host cell according to Claim 18 wherein a second recombinant DNA compound comprises, or the first recombinant DNA compound further comprises, the compound of any one of Claims 6 to 10 encoding for the heavy chain of the chimeric antibody, and transcriptional and translational DNA sequences positioned in relation to the heavy chain-encoding DNA strand sequence to direct expression of the heavy chain.

20. A chimeric monoclonal antibody comprising a chelate-specific light chain variable region derived from a first mammalian species and a constant region derived from a second and different mammalian species, the variable light chain region having an amino acid sequence comprising:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly.

21. A chimeric monoclonal antibody comprising a chelate-specific heavy chain variable region derived from a first mammalian species and a constant region derived from a second and different mammalian species, the variable heavy chain region having an amino acid sequence comprising:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

22. A chimeric monoclonal antibody comprising a light chain variable region having the amino acid sequence of Claim 20 and a heavy chain variable region having the amino acid sequence of Claim 21.

23. The chimeric monoclonal antibody according to Claim 20, 21 or 22 wherein the variable region of the chain is derived from a murine hybridoma.

24. The chimeric monoclonal antibody according to Claim 23 wherein the murine hybridoma is CHA 255.5.

25. The chimeric monoclonal antibody according to any of Claims 20 to 24 wherein the constant region of the antibody is derived from a human lymphocyte.

Claims for the following Contracting State: GR

1. A recombinant DNA compound comprising a first DNA sequence which encodes for the chelate-specific light chain variable region of a chimeric monoclonal antibody, the first DNA sequence coding for an amino acid sequence comprising:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly.

2. The recombinant DNA compound according to Claim 1 wherein the first DNA strand coding sequence comprises:

CAG GCT GTT GTG ACT CAG GAA TCT GCA CTC ACC ACA TCA CCT GGT GAA ACA GTC ACA CTC ACT TGT CGC TCA AGT ACT GGG GCT GTT ACA ACT AGT AAC TAT GCC AAC TGG GTC CAA GAA AAA CCA GAT CAT TTA TTC ACT GGT CTA ATA GGT GGT ACC AAT AAC CGG GCT CCA GGT GTT CCT GCC AGA TTC TCA GGC TCC CTG ATT GGA GAC AAG GCT GCC CTC ACC ATC ACA GGG GCA CAG ACT GAA GAT GAG GCA AGA TAT TTC TGT GCT CTA TGG TAC AGC AAC CTC TGG GTA TTC GGT GGA GGA ACC AAA CTG ACT GTC CTA CTA GGG.

3. The recombinant DNA compound according to Claim 1 or 2 wherein the first DNA coding sequence further comprises a DNA sequence coding for a eukaryotic signal peptide.

4. The recombinant DNA compound according to Claim 1, 2 or 3 wherein the DNA construct further comprises a second DNA strand sequence which codes for the light chain constant region of the chimeric monoclonal antibody.

5. The recombinant DNA compound according to Claim 3 wherein the DNA strand sequence coding for the signal peptide comprises:

ATG GCC TGG ATT TCA CTT ATA CTC TCT CTC CTG GCT CTC AGC TCA GGT GCC ATT TCC.

6. A recombinant DNA compound comprising a first DNA sequence which encodes for the chelate-specific heavy chain variable region of a chimeric monoclonal antibody, the first DNA sequence coding for an amino acid sequence comprising:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala.

7. The recombinant DNA compound according to Claim 6 wherein the first DNA strand coding sequence comprises:

GAA GTG ACG CTG GTG GAG TCT GGG GGA GAC TCA GTG AAG CCT GGA GGG TCC CTG AAA CTC TCC TGT GCA GCC TCT GGA TTC ACT TTA AGT GGT GAA ACC ATG TCT TGG GTT CGC CAG ACT CCG GAG AAG AGG CTG GAG TGG GTC GCA ACC ACT CTT AGT GGT GGT GGT TTC ACC TTC TAT TCA GCC AGT GTG AAG GGT CGT TTC ACC ATC TCC AGA GAC AAT GCC CAG AAC AAC CTC TAT CTA CAA CTG AAT AGT CTG AGG TCT GAG GAC ACG GCC TTG TAT TTC TGT GCA AGT CAT CGG TTT GTT CAC TGG GGC CAC GGG ACT CTG GTC ACT GTC TCT GCA GCC.

8. The recombinant DNA compound according to Claim 6 or 7 wherein the first DNA coding sequence further comprises a DNA sequence for a eukaryotic signal peptide.

9. The recombinant DNA compound according to Claim 6, 7 or 8 wherein the DNA construct further comprises a second DNA strand sequence which codes for the heavy chain constant region of the chimeric monoclonal antibody.

10. The recombinant DNA compound according to Claim 8 wherein the DNA strand sequence coding for the signal peptide comprises:

ATG AGC TTT GGG CTG AGC TTG ATT TTC CTT GTC CTA ATT TTA AAA GGT GTC CAG TGT.

11. The recombinant DNA compound according to any of Claims 1 to 10 wherein the first DNA strand coding sequence is derived from a murine hybridoma.

12. The recombinant DNA compound according to Claim 11 wherein the murine hybridoma is CHA 255.5.

13. The recombinant DNA compound according to Claim 4 or 8 wherein the second DNA strand coding sequence is derived from a human lymphocyte.

14. The recombinant DNA compound of Claim 1 or 2 that is plasmid pMLCH-1 as shown in Figure 1 or plasmid pMLCH1dB as shown in Figure 1.

15. The recombinant DNA compound of Claim 6 or 7 that is plasmid pUCVHInc-1A as shown in Figure 3.

16. The recombinant DNA compound of Claim 4 that is plasmid pGCHAK as shown in Figure 1.

17. The recombinant DNA compound of Claim 8 that is plasmid pHG1-CHA as shown in Figure 4 or plasmid pNCHAG1 as shown in Figure 4.

18. A eukaryotic host cell capable of expression of a chimeric monoclonal antibody comprising the recombinant DNA compound of any of Claims 1 to 5 encoding for the light chain of the chimeric antibody, and transcriptional and translational DNA sequences positioned in relation to the light chain-encoding DNA sequence to direct expression of the light chain.

19. The eukaryotic host cell according to Claim 18 wherein a second recombinant DNA compound comprises, or the first recombinant DNA compound further comprises, the compound of any one of Claims 6 to 10 encoding for the heavy chain of the chimeric antibody, and transcriptional and translational DNA sequences positioned in relation to the heavy chain-encoding DNA strand sequence to direct expression of the heavy chain.

Claims for the following Contracting State: ES

1. A method of expressing chimeric antibody chains in transfected cells, said antibody chains comprising variable regions which bind to metal chelates, said method comprising the steps of:
a) constructing a recombinant DNA vector which comprises a gene encoding a light chain variable region with the amino acid sequence:
Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly,
b) transfecting said vector into a mammalian host cell, and
c) culturing said host cell under conditions suitable for the expression of said variable region gene.

2. The method of Claim 1 wherein the recombinant DNA vector is plasmid pGCHAK as shown in Figure 1.

3. The method of Claim 2 wherein the transfected cell is an SP2/0 cell.

4. A method of expressing chimeric antibody chains in transfected cells, said antibody chains comprising variable regions which bind to metal chelates, said method comprising the steps of:
a) constructing a recombinant DNA vector which comprises a gene encoding a heavy chain variable

31

region with the amino acid sequence:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Tyr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala,

b) transfecting said vector into a mammalian host cell, and

c) culturing said host cell under conditions suitable for the expression of said variable region.

5. The method of Claim 3 wherein the recombinant DNA vector is plasmid pNCHAG1 as shown in Figure 4.

6. The method of Claim 2 wherein the transfected cell is an SP2/0 cell.

7. A method of expressing chimeric antibodies in transfected cells, said antibodies comprising variable regions which bind to metal chelates, said method comprising the steps of:

a) constructing a recombinant DNA encoding a light chain variable region with the amino acid sequence:

Gln Ala Val Val Thr Gln Glu Ser Ala Leu Thr Thr Ser Pro Gly Glu Thr Val Thr Leu Thr Cys Arg Ser Ser Thr Gly Ala Val Thr Thr Ser Asn Tyr Ala Asn Trp Val Gln Glu Lys Pro Asp His Leu Phe Thr Gly Leu Ile Gly Gly Thr Asn Asn Arg Ala Pro Gly Val Pro Ala Arg Phe Ser Gly Ser Leu Ile Gly Asp Lys Ala Ala Leu Thr Ile Thr Gly Ala Gln Thr Glu Asp Glu Ala Arg Tyr Phe Cys Ala Leu Trp Tyr Ser Asn Leu Trp Val Phe Gly Gly Gly Thr Lys Leu Thr Val Leu Gly

and

b) constructing a recombinant DNA vector which comprises a gene encoding a heavy chain variable region with the amino sequence:

Glu Val Thr Leu Val Glu Ser Gly Gly Asp Ser Val Lys Pro Gly Gly Ser Leu Lys Leu Ser Cys Ala Ala Ser Gly Phe Thr Leu Ser Gly Glu Thr Met Ser Trp Val Arg Gln Thr Pro Glu Lys Arg Leu Glu Trp Val Ala Thr Thr Leu Ser Gly Gly Gly Phe Thr Phe Tyr Ser Ala Ser Val Lys Gly Arg Phe Thr Ile Ser Arg Asp Asn Ala Gln Asn Asn Leu Tyr Leu Gln Leu Asn Ser Leu Arg Ser Glu Asp Thr Ala Leu Tyr Phe Cys Ala Ser His Arg Phe Val His Trp Gly His Gly Thr Leu Val Thr Val Ser Ala Ala,

or

c) constructing a recombinant DNA vector which comprises said gene encoding a light chain variable region and said gene encoding a heavy chain variable region, and

d) transfecting said vectors a) and b) or said vector c) into a mammalian host cell, and

e) culturing said host cell under conditions suitable for the expression of said variable region genes.

8. The method of Claim 7 wherein the plasmid of a) is plasmid pGCHAK and the plasmid of b) is plasmid pNCHAG1.

9. The method of Claim 8 wherein the transfected cell is an SP2/0 cell.

Neu einge
Nouve

# FIG.I
# Restriction Site and Function Maps of Plasmids
# pMLCH1 and pGCHAK

# FIG.2
## Restriction Site and Function Map of Plasmid pHKF-1

# FIG.3
## Restriction Site and Function Maps of Plasmids pUCVHInc-1A and pHG1Z

pUCVHInc-1A

pHG1Z

Ne

# FIG. 4
# Restriction Site and Function Maps of Plasmids pHG1-CHA and pNCHAG1

**pHG1-CHA** (14.4 kb) — Cla I, Eco RI, Hind III, CHA V$_H$ J$_H$, Xba I, Eco RI, Xba I, Hind III, Human Gamma I, Human Gamma I, Bam HI, pBR 322, Amp$^r$

**pNCHAG1** (15.4 kb) — Cla I, Eco RI, Hind III, CHA V$_H$, Xba I, Eco RI, Xba I, Hind III, Human C Gamma, Bam HI, Hind III, Amp$^r$, neo SV40 ori

Nε

# FIG. 5
## The Construction of P19HANCH Vector

# FIG. 6
## Restriction Site and Function Map of Plasmid pGCHAK-3